(19) Europäisches Patentamt
European Patent Office
Office européen des brevets

(11) Numéro de publication : 0 234 970 B1

# (12) FASCICULE DE BREVET EUROPEEN

(45) Date de publication du fascicule du brevet : **17.04.91 Bulletin 91/16**

(51) Int. Cl.$^5$ : **C07D 471/04,** A61K 31/435, A61K 31/55, // (C07D471/04, 235:00, 221:00)

(21) Numéro de dépôt : **87400075.5**

(22) Date de dépôt : **14.01.87**

(54) **Dérivés d'acylaminométhyl-3 imidazo[1,2-a]pyridines, leur préparation et leur application en thérapeutique.**

Le dossier contient des informations techniques présentées postérieurement au dépôt de la demande et ne figurant pas dans le présent fascicule.

(30) Priorité : **22.01.86 FR 8600836**
**05.02.86 FR 8601553**

(43) Date de publication de la demande : **02.09.87 Bulletin 87/36**

(45) Mention de la délivrance du brevet : **17.04.91 Bulletin 91/16**

(84) Etats contractants désignés : **AT BE CH DE ES FR GB GR IT LI LU NL SE**

(56) Documents cités :
**EP-A- 033 094**
**EP-A- 050 563**
**EP-A- 0 172 096**
**GB-A- 991 589**
**GB-A- 1 076 589**
**J.Med. Chem. 12, 122(1969)**

(73) Titulaire : **SYNTHELABO**
**58 rue de la Glacière**
**F-75013 Paris (FR)**

(72) Inventeur : **George, Pascal**
**39, rue Henri de Vilmorin**
**F-94400 Vitry sur Seine (FR)**
Inventeur : **Giron, Claudie**
**1, Parvis de la Bièvre Appt. 76**
**F-92160 Antony (FR)**
Inventeur : **Froissant, Jacques**
**Cédex 981**
**F-41160 Moree Brevainville (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**

**EP 0 234 970 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés d'acylaminométhyl-3 imidazo[1,2-a]pyridines, leur préparation et leur application en thérapeutique.

Les composés de l'invention répondent à la formule (I) donnée dans le schéma donné ci-après, formule dans laquelle

$R_1$ représente un atome d'hydrogène ou un groupe alkyle en $C_1$-$C_4$ linéaire ou ramifié, ou un groupe benzyle,

$R_2$ représente un groupe alkyle en $C_1$-$C_6$ linéaire ou ramifié, un groupe cyclohexyle, trichlorométhyle, propène-1 yle, allyle, phényle, chloro-4 phényle ou benzyle,

ou bien encore

$R_1$ et $R_2$ représentent ensemble une chaîne aliphatique en $C_3$-$C_5$,

et, ou bien,

a) $X_1$ et $X_2$ représentent chacun un atome d'hydrogène, $X_3$ représente un atome d'halogène ou un groupe alkyle en $C_1$-$C_3$, un groupe méthylthio, un groupe trifluorométhyle, un groupe carboxy éventuellement estérifié (de formule COOR dans laquelle R représente l'hydrogène ou un alkyle en $C_1$-$C_6$), un groupe cyano, un groupe aminocarbonyle éventuellement monoou di-alkylé (de formule $CONR_3R_4$ dans laquelle $R_3$ et $R_4$, indépendamment l'un de l'autre, représentent chacun l'hydrogène ou un alkyle en $C_1$-$C_4$ ou représentent ensemble une chaîne de formule $(CH_2)_2$-Z-$(CH_2)_2$ dans laquelle Z représente une liaison ou un atome d'oxygène ou de soufre ou un groupe de formule $CH_2$, NH ou N(alkyle en $C_1$-$C_4$)), un groupe alkylamino (de formule $NHR_5$ dans laquelle $R_5$ représente un alkyle en $C_2$-$C_6$) ou dialkylamino (de formule $NR_6R_7$ dans laquelle $R_6$ et $R_7$, indépendamment l'un de l'autre, représentent chacun un alkyle en $C_2$-$C_6$ ou représentent ensemble une chaîne de formule $(CH_2)_2$-Z-$(CH_2)_2$ dans laquelle Z représente une liaison ou un atome d'oxygène ou de soufre ou un groupe de formule $CH_2$, NH ou N(alkyle en $C_1$-$C_4$)), et

Y représente un groupe méthyle, un groupe trifluorométhyle, un groupe carboxy éventuellement estérifié (de formule $COOR_8$ dans laquelle $R_8$ représente l'hydrogène ou un alkyle en $C_1$-$C_6$), un groupe cyano, un groupe aminocarbonyle éventuellement mono- ou di-alkylé (de formule $CONR_9R_{10}$ dans laquelle $R_9$ et $R_{10}$, indépendamment l'un de l'autre, représentent chacun l'hydrogène ou un alkyle en $C_1$-$C_4$), ou un groupe amino éventuellement mono- ou dialkylé (de formule $NR_{11}R_{12}$ dans laquelle $R_{11}$ et $R_{12}$, indépendamment l'un de l'autre, représentent chacun l'hydrogène ou un alkyle en $C_1$-$C_4$), étant exclus dans le cas (a) les composés de formule (I) dans laquelle $X_3$ représente un atome d'halogène ou un groupe alkyle ou méthylthio et Y représente un groupe méthyle,

ou bien

b) $X_1$, $X_2$ et $X_3$ représentent chacun, indépendamment l'un de l'autre, l'hydrogène, un halogène ou un groupe alkyle en $C_1$-$C_4$, méthoxy, méthylthio, éthylthio, méthylsulfonyle, nitro, amino, méthylamino, diméthylamino, acétylamino ou diacétylamino, et

Y représente un groupe méthyle, étant exclus dans le cas (b) les composés dans la formule (I) desquels $X_1$ et $X_2$ représentent chacun un atome d'hydrogène.

Des composés ayant une structure chimique analogue à celle des composés de l'invention sont décrits dans les demandes de brevets GB-991589, GB-1076089, EP-0050563, EP-0033094 et dans J. Med. Chem., 12, (1969), 122. La demande de brevet EP-0050563 décrit également des applications thérapeutiques semblables à celles des composés de l'invention.

Les composés de l'invention peuvent se présenter à l'état de bases libres ou de sels d'addition à des acides acceptables en pharmacologie.

Les composés préférés sont ceux dans la formule desquels $R_1$ représente un atome d'hydrogène ou un groupe méthyle, et $R_2$ représente un groupe propyle ou isobutyle.

Les composés de l'invention peuvent être préparés conformément au schéma de la page suivante.

L'amino-2 pyridine de formule (II) portant un substituant Y est mise en réaction avec une bromo-2 éthanone de formule (III) portant les substituants $X_1$, $X_2$ et $X_3$, dans un solvant protique tel qu'un alcool aliphatique, à chaud, en présence ou non d'une base telle qu'un carbonate ou hydrogénocarbonate alcalin. L'imidazo[1,2-a]pyridine de formule (IV) ainsi

Schéma

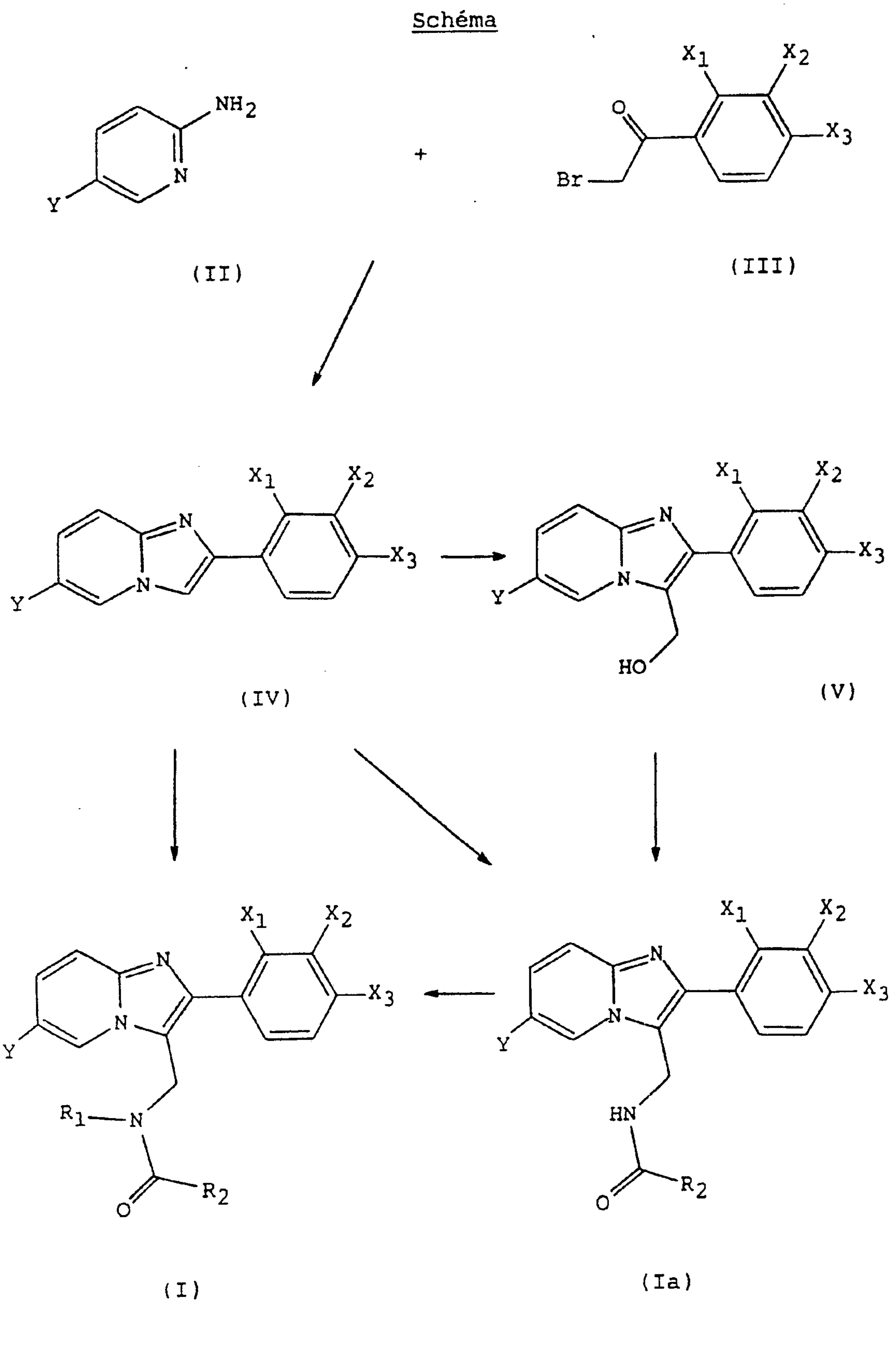

(II) (III) (IV) (V) (I) (Ia)

obtenue est hydroxyméthylée au moyen de formol dans un solvant du type acide carboxylique tel que l'acide acétique, ou tout autre moyen équivalent. On obtient ainsi l'alcool de formule (V).

Cet alcool (V) peut être également obtenu à partir de l'imidazo[1,2-a]pyridine de formule (IV) en deux étapes, la première étant une formylation, par exemple au moyen du réactif que l'on obtient par action du chlorure d'oxalyle sur le diméthylformamide. On obtient après hydrolyse un aldéhyde que, dans la deuxième étape, on réduit en alcool correspondant, de formule (V), de manière connue, par exemple par action d'un borohydrure de métal alcalin.

L'alcool (V) est ensuite mis à réagir avec un nitrile $R_2$-CN en milieu acide, par exemple acétique ou sulfurique, à une température de 0 à 150°C, suivant le nitrile utilisé. On obtient ainsi après hydrolyse un composé de formule (Ia) dont le substituant $R_1$ est nécessairement l'hydrogène. Si on le désire, on peut alors alkyler ou benzyler ce composé de manière connue, par exemple avec un halogénure de formule $R_1$-hal en présence d'un hydrure de métal alcalin et dans un solvant approprié, tel que le tétrahydrofuranne, pour aboutir à un composé de formule (I) dans laquelle $R_1$ est un groupe alkyle ou benzyle.

On peut également préparer les composés de formule (I) en deux étapes seulement, en faisant réagir l'imidazopyridine (IV) avec un amide de formule

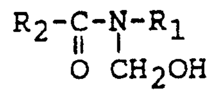

dans laquelle $R_1$ et $R_2$ sont tels que définis ci-dessus. La réaction s'effectue à la température ambiante, en présence d'acide sulfurique concentré et éventuellement avec un cosolvant tel que l'acide acétique glacial.

Enfin on peut encore préparer des composés de formule (Ia) en faisant réagir l'imidazopyridine (IV) avec un nitrile de formule $R_2$-CN et le paraformaldéhyde, à chaud, en milieu acétique et en présence d'acide sulfurique.

Le composé (I) obtenu après hydrolyse peut, si on le désire, être ensuite alkylé ou benzylé comme indiqué plus haut.

Les composés de formule (I) où $X_1$, $X_2$, $X_3$ et/ou Y représentent un groupe amino peuvent être obtenus par réduction des composés nitrés correspondants au moyen d'un métal tel que le fer ou le zinc dans un solvant protique ou acide tel que l'éthanol ou l'acide acétique, ou encore par des dérivés d'étain tels que le chlorure stanneux.

Les composés de formule (I) où $X_1$, $X_2$, $X_3$ et/ou Y représentent un groupe alkylamino ou dialkylamino peuvent être obtenus, soit par alkylation de l'amine primaire au moyen d'un halogénure d'alkyle en présence de base tertiaire, soit par amination réductrice au moyen d'aldéhyde et de borohydrure alcalin en milieu protique neutre ou acide.

Il va de soi que ces dérivés aminés peuvent être obtenus directement à partir des composés de formules (II) et (III) où $X_3 = NHR_5$ ou $NR_6R_7$ et $Y = NR_{11}R_{12}$ dès le départ, comme le précise le schéma de la synthèse.

Les composés de formule (I) où $X_3$ et/ou Y représentent un groupe cyano peuvent être obtenus par réaction des composés de formule (I), où $X_3$ et/ou Y représentent un atome d'halogène, particulièrement l'iode, avec un cyanure tel que le cyanure cuivreux dans un solvant organique tel que la pyridine ou le diméthylformamide. Ils peuvent aussi être obtenus directement à partir des composés de formules (II) et (III) où $X_3$ et/ou Y représentent un groupe cyano dès le départ, comme le précise le schéma de synthèse.

Les composés de formule (I) où $X_3$ et/ou Y représentent un groupe carboxylate (de formule $CO_2R$ ou $COOR_8$, respectivement) peuvent être obtenus par réaction des composés de formule (I), où $X_3$ et/ou Y représentent un groupe cyano, avec l'acide chlorhydrique gazeux dans l'alcool correspondant ROH ou $R_8OH$ comme solvant, puis hydrolyse. Ils peuvent être aussi obtenus directement à partir des composés de formules (II) et (III) où $X_3$ et/ou Y représentent un groupe carboxylate dès le départ, comme le précise le schéma de synthèse.

Les composés de formule (I) où $X_3$ et/ou Y représentent un groupe carboxyle peuvent être obtenus par hydrolyse acide des composés correspondant de formule (I) où $X_3$ et/ou Y représentent un groupe cyano. L'hydrolyse peut être réalisée par exemple en milieu acide chlorhydrique, à chaud, seul ou avec un cosolvant organique acide tel que l'acide acétique.

Les composés de formule (I) où $X_3$ représente un groupe $CONR_3R_4$ et/ou Y représentent un groupe $CONR_9R_{10}$ peuvent être obtenus classiquement par amidification des acides correspondants, par exemple par réaction des composés de formule (I) où $X_3$ et/ou Y représentent un groupe de formule $CO_2H$ avec du carbonyldiimidazole dans un solvant inerte tel que le tétrahydrofuranne, suivie d'une aminolyse de l'imidazolide intermédiaire obtenu in situ au moyen d'une amine de formule $R_3R_4NH$ ou $R_9R_{10}NH$, respectivement, dont les substituants $R_3$, $R_4$, $R_9$ et $R_{10}$ répondent aux définitions données ci-dessus.

Plus particulièrement, les composés de formule (I) où $X_3$ et/ou Y représentent un groupe $CONH_2$ peuvent

aussi être préparés soit par hydrolyse partielle des nitriles correspondants, soit par réaction des mêmes nitriles avec l'acide chlorhydrique gazeux dans l'acide formique.

Les exemples qui vont suivre illustrent la préparation de quelques composés selon l'invention. Les microanalyses et les spectres IR et RMN ont confirmé les structures des produits obtenus.

Exemple 1

N-[[(méthyl-4 phényl)-2 trifluorométhyl-6 imidazo[1,2-a]pyridinyl-3]méthyl]-N,3-diméthyl-butanamide.

1.1. (Méthyl-4 phényl)-2 trifluorométhyl-6 imidazo[1,2-a] pyridine.

On mélange 10 g (0,0616 mole) d'amino-2 trifluorométhyl-5 pyridine et 17,5 g (0,0616 mole) de bromo-1 (méthyl-4 phényl)-2 éthanone-2 dans 200 ml de n-propanol et chauffe la solution à reflux. En fin de réaction, on concentre le mélange réactionnel sous pression réduite, on reprend le résidu d'évaporation avec de l'eau et on le traite par un excès d'ammoniaque jusqu'à pH basique. On extrait l'imidazopyridine au dichlorométhane et on la purifie par chromatographie. On obtient 11,95 g (56%) de solide blanc.
F = 187-188°C.

1.2. (Méthyl-4 phényl)-2 trifluorométhyl-6 imidazo[1,2-a] pyridine-3-méthanol.

On dissout dans 160 ml d'acide acétique 13,8 g (0,0463 mole) d'imidazopyridine préparée selon 1.1. et 32 ml de formol à 37% dans l'eau. On maintient cette solution à 60°C pendant 3 heures puis on classe le solvant sous pression réduite. On reprend le résidu d'évaporation par 70 ml d'eau, 21 ml de lessive de soude et 260 ml de dichlorométhane. On agite le mélange biphasique pendant 1 heure puis on sépare l'insoluble par filtration, on le lave à l'acétone puis à l'éther et on le sèche. On obtient 6,17 g de l'alcool attendu (44%).
F = 216-218°C.

1.3. N-[[(méthyl-4 phényl)-2 trifluorométhyl-6 imidazo[1,2-a] pyridinyl-3]méthyl]méthyl-3 butanamide.

Dans un ballon de 250 ml, on introduit 6,1 g (0,02 mole) de l'alcool obtenu selon 1.2. et 40 ml d'isovaléronitrile. On chauffe la suspension à 100°C et on y ajoute goutte à goutte 5,7 ml d'acide sulfurique concentré. Tous les réactifs passent en solution, puis un précipité apparaît. On ajoute ensuite de la glace et dilue le milieu à l'eau, traite à l'ammoniaque (jusqu'à pH > 8) et extrait l'amide au dichlorométhane.
On recueille finalement 5,9 g (80%) de produit.
F = 212-213°C.

1.4. N-[[(méthyl-4 phényl)-2 trifluorométhyl-6 imidazo[1,2-a] pyridinyl-3]méthyl]-N,3-diméthyl-butanamide.

On met en suspension 1,23 g (0,0256 mole) d'hydrure de sodium à 50% dans l'huile dans 40 ml de tétrahydrofuranne avec 2 ml de diméthylformamide. On y ajoute une solution de 60 ml de tétrahydrofuranne et 3 ml de diméthylformamide contenant 5 g (0,0128 mole) d'amide préparé selon 1.3. et 1,6 ml d'iodure de méthyle. On agite le mélange jusqu'à fin de dégagement gazeux puis encore pendant 1 heure, et on le concentre sous pression réduite. On reprend le résidu d'évaporation avec de l'eau et on extrait l'amide tertiaire au dichlorométhane. Après séchage on obtient 5 g (96%) d'amide.
F = 196-197°C.

Exemple 2

N-[[(méthyl-4 phényl)-2 nitro-6 imidazo[1,2-a]pyridinyl-3] méthyl]-N,3-diméthyl-butanamide.

2.1. (Méthyl-4 phényl)-2 nitro-6 imidazo[1,2-a]pyridine.

On met en réaction dans 900 ml de n-propanol 81 g (0,38 mole) de bromo-1 (méthyl-4 phényl)-2 éthanone-2 et 53 g (0,38 mole) de amino-2 nitro-5 pyridine. On maintient la solution au reflux, en suivant la réaction par chromatographie sur couche mince. Lorsqu'il n'y a plus d'évolution, on évapore le mélange à sec. On reprend le résidu avec de l'eau et on le traite avec de l'ammoniaque jusqu'à pH > 8. On filtre l'insoluble, on le sèche puis on le traite au dichlorométhane. On recristallise dans l'acétate d'éthyle la partie insoluble, ce qui fournit un premier jet. Ensuite on rassemble les eaux mères avec la partie soluble dans le dichlorométhane, on concen-

tre et on obtient un deuxième jet de produit. On purifie les deux jets séparément par chromatographie. On obtient globalement 22 g (23%) de solide jaune.
F = 205-206°C.

2.2. (Méthyl-4 phényl)-2 nitro-6 imidazo[1,2-a]pyridine-3-méthanol.

A 200 ml d'acide acétique, on ajoute successivement 14 g (0,056 mole) d'imidazopyridine préparée selon 2.1. et 45 ml de formol aqueux à 37%. On chauffe la solution à 60°C pendant 4 heures. En fin de réaction, on évapore le solvant sous pression réduite et on traite le résidu solide par 76 ml d'eau, 25 ml de lèssive de soude et 310 ml de dichlorométhane. On agite ce mélange biphasique pendant 1/2 heure puis on le traite par un excès d'ammoniaque et on l'agite encore pendant 1/2 heure. On recueille l'insoluble par filtration et on le lave à l'eau, guis à l'acétate d'éthyle et à l'éther. On obtient 11,5 g (73%) d'alcool.
F = 236-238°C.

2.3. N-[[(méthyl-4 phényl)-2 nitro-6 imidazo[1,2-a]pyridinyl-3]méthyl]méthyl-3 butanamide.

On introduit dans un ballon de 100 ml, 6,88 g (0,0242 mole) d'alcool préparé selon 2.2., 40 ml d'isovaléronitrile et on porte la solution à 100°C. On ajoute à cette suspension, goutte à goutte, 6,7 ml d'acide sulfurique et laisse le mélange sous agitation jusqu'à obtention de 2 phases. On élimine la phase supérieure et on traite le reste du mélange réactionnel avec de la glace puis on le dilue à l'eau et on le traite par de l'ammoniaque concentrée jusqu'à pH > 8. On filtre l'insoluble, on le lave au dichlorométhane puis à l'eau, à l'acétone et enfin à l'éther.
On obtient 6,29 g de produit pur.
On récupère 2,24 g par évaporation partielle du dichlorométhane. On rassemble les deux lots et recristallise le produit dans l'acétate d'éthyle.
F = 239-241°C.

Exemple 3

N-[[(méthyl-4 phényl)-2 amino-6 imidazo[1,2-a]pyridinyl-3] méthyl]méthyl-3 butanamide.

Dans 25 ml d'éthanol on introduit 1 g (0,0026 mole) d'amide préparé selon 2.3. et 2,5 g de chlorure stanneux. On chauffe le mélange à 70°C pendant 3 h, puis on l'évapore à sec. On reprend le résidu avec de l'eau et de l'ammoniaque 1 N et on extrait le produit au dichlorométhane. On sèche la phase organique sur sulfate de sodium, on la filtre, on l'évapore sous pression réduite. On purifie le résidu d'évaporation par chromatographie sur colonne.
F = 245-247°C.

Exemple 4

N-[[méthyl-6 (trifluorométhyl-4 phényl)-2 imidazo[1,2-a]pyridinyl-3]méthyl]-N,3-diméthyl-butanamide.

4.1. Méthyl-6 (trifluorométhyl-4 phényl)-2 imidazo[1,2-a] pyridine.

On mélange dans 210 ml d'éthanol à 95%, 24 g (0,077 mole) de bromo-1 (trifluorométhyl-4 phényl)-2 éthanone-2, 8,4 g (0,077 mole) de amino-2 méthyl-5 pyridine et 13 g de bicarbonate de sodium et chauffe cette suspension à reflux jusqu'à fin de dégagement gazeux. On évapore ensuite le solvant sous pression réduite, on reprend le résidu d'évaporation avec de l'eau et du dichlorométhane. On décante la phase organique, on la sèche sur sulfate de magnésium et on élimine le solvant par évaporation sous pression réduite. On purifie le résidu par chromatographie sur colonne.
On obtient 18,4 g (78%) d'imidazopyridine.
F = 216-217°C.

4.2. Méthyl-6 (trifluorométhyl-4 phényl)-2 imidazo[1,2-a] pyridine-3-méthanol.

Dans 230 ml d'acide acétique, on dissout 18 g (0,065 mole) d'imidazopyridine obtenue selon 4.1. et 46 ml de formol à 37% dans l'eau. On maintient à 60°C jusqu'à disparition du produit de départ (suivi par chromatographie sur couche mince) puis on le concentre sous pression réduite. On reprend le résidu avec 90 ml d'eau,

29 ml de lessive de soude et 360 ml de dichlorométhane et on l'agite pendant 1/4 d'heure. On collecte l'insoluble par filtration et on le lave à l'eau, à l'acétone et à l'éther. On obtient 14 g (70%) d'alcool.
F = 225-226°C.

4.3. N-[[méthyl-6 (trifluorométhyl-4 phényl)-2 imidazo[1,2-a] pyridinyl-3]méthyl]méthyl-3 butanamide.

Dans un ballon de 250 ml, on introduit 7 g (0,0228 mole) d'alcool obtenu selon 4.2. et 40 ml d'isovaléronitrile. On chauffe cette suspension à 100°C et on y ajoute goutte à goutte 6,5 ml d'acide sulfurique. Tous les réactifs passent en solution, puis un précipité apparaît. On dilue le milieu avec de la glace, puis de l'eau, et ensuite traite la solution par de l'ammoniaque jusqu'à pH > 8 et enfin par 200 ml de dichlorométhane. On décante la phase organique, on la sèche sur sulfate de sodim.
Après filtration et évaporation du solvant, on obtient 8 g (90%) d'amide.
F = 200-202°C.

4.4. N[[méthyl-6 (trifluorométhyl-4 phényll-2 imidazo[1,2-a] pyridinyl-3]méthyl]-N,3-diméthyl-butanamide.

On ajoute à une suspension de 1,23 g (0,0256 mole) d'hydrure de sodium à 50% dans l'huile dans un mélange de 40 ml de tétrahydrofurane et 2 ml de diméthylformamide, 5 g (0,128 mole) de l'amide secondaire obtenu selon 4.3. et 1,6 ml d'iodure de méthyle dissous dans 63 ml d'un mélange de tétrahydrofuranne/diméthylformamide (95/5). Après la fin du dégagement gazeux, on maintient l'agitation pendant 1 heure puis on ajoute 1 ml de méthanol. On évapore le solvant sous pression réduite, on reprend le résidu d'évaporation à l'eau et on extrait l'amide tertiaire au dichlorométhane.
On obtient 4,5 g (87%) d'amide tertiaire.
F = 129-130°C.

Exemple 5

[Méthyl-6 [(méthyl-3 butanoyl)aminométhyl]-3 imidazo[1,2-a] pyridinyl-2]-4 benzoate d'éthyle.

5.1. (Méthyl-6 imidazo[1,2-a]pyridinyl-2)-4 benzoate d'éthyle.

Dans 1 l d'éthanol, on introduit 67 g (0,62 mole) de amino-2 méthyl-5 pyridine, 86 g (1,02 mole) de bicarbonate de sodium et 143 g (0,527 mole) de (bromo-2 acétyl)-4 benzoate d'éthyle. On porte le mélange à reflux pendant 3 heures, laisse au repos 19 heures, récupère par filtration le solide formé, le lave abondamment à l'eau et le sèche. On le recristallise dans un mélange de dichlorométhane/méthanol.
F = 228-230°C.

5.2. (Hydroxyméthyl-3 méthyl-6 imidazo[1,2a]pyridinyl-2)-4 benzoate d'éthyle.

A 200 ml d'acide acétique, on ajoute successivement 31 g (0,1 mole) du benzoate obtenu selon 5.1. et 81 g (1 mole) de formol à 37% dans l'eau. On maintient l'agitation plusieurs heures puis on évapore l'acide acétique. On reprend le résidu d'évaporation avec de l'eau puis avec un excès d'ammoniaque jusqu'à pH basique. On extrait le solide au dichlorométhane et le recristallise dans un mélange dichlorométhane/éther. F = 157-159°C.

5.3. [Méthyl-6[(méthyl-3 butanoyl)aminométhyl]-3 imidazo [1,2-a]pyridinyl-2]-4 benzoate d'éthyle.

On mélange 20 ml d'isovaléronitrile et 5 g (0,016 mole) d'alcool préparé selon 5.2. puis ajoute goutte à goutte 4 ml d'acide sulfurique concentré et chauffe le milieu réactionnel à 100°C jusqu'à ce que 2 phases se forment. On traite la phase inférieure par 100 g de glace, on la dilue à l'eau ou la traite à l'ammoniaque jusqu'à pH basique. On extrait l'amide au dichlorométhane et on le purifie par chromatographie. On obtient un solide blanc.
F = 230-231°C.

Exemple 6

N-[[méthyl-6[(pipéridinyl-1)-4 phényl]-2 imidazo[1,2-a]pyridinyl-3]méthyl]-N,3-diméthyl-butanamide.

6.1. Méthyl-6 (nitro-4 phényl)-2 imidazo[1,2-a]pyridine.

On met en réaction, dans 200 ml d'éthanol à 95%, 59,5 g (0,2 mole) de bromo-1 (nitro-4 phényl)-2 éthanone-2, 21,6 g (0,2 mole) de amino-2 méthyl-5 pyridine et 34 g de bicarbonate de sodium. On maintient l'agitation et le reflux pendant 3 heures puis on refroidit le mélange réactionnel et on évapore l'éthanol sous pression réduite. On agite le résidu d'évaporation dans 700 ml d'eau à 70°C pendant 3 heures, puis on filtre l'insoluble, on le lave avec 50 ml d'éthanol puis a l'éther et on le sèche.
On obtient 43,6 g d'imidazopyridine.
F = 237-239°C.

6.2. Méthyl-6 (nitro-4 phényl)-2 imidazo[1,2-a]pyridine-3-méthanol.

Dans 700 ml d'acide acétique glacial, on mélange 50 g (0,2 mole) d'imidazopyridine obtenue selon 6.1. et 140 ml de formol aqueux à 37% et on porte la solution à 60°C pendant 3 heures. On évapore ensuite le solvant sous pression réduite et on reprend le résidu d'évaporation avec de l'eau, on le traite à l'ammoniaque jusqu'à pH > 8, et on le reprend entre l'eau et le dichlorométhane. On sépare l'insoluble par filtration et on le lave à l'éther. On le recristallise dans un mélange de méthanol et d'éthanol.
On obtient 36 g (64%) de produit.
F = 237-238°C.

6.3. N-[[méthyl-6 (nitro-4 phényl)-2 imidazo[1,2-a]pyridinyl-3]méthyl]méthyl-3 butanamide.

Dans 20 ml d'isovaléronitrile contenant 2 g (0,0076 mole) d'alcool obtenu selon 6.2., on introduit goutte à goutte sous agitation 0,8 ml d'acide sulfurique concentré. Puis on chauffe la suspension jusqu'à ce qu'il se forme deux phases. On élimine le liquide surnageant, on hydrolyse le reste du mélange puis on le traite par un excès d'ammoniaque jusqu'à pH > 8. On extrait l'insoluble au dichlorométhane ; après séchage, filtration et évaporation du solvant, on obtient 9,10 g (88%) d'amide secondaire.
F = 221-22°C.

6.4. N-[[méthyl-6 (nitro-4 phényl)-2 imidazo[1,2-a]pyridinyl-3]méthyl]-N,3-diméthyl-butanamide.

A une suspension de 1,83 g (0,076 mole) d'hydrure de sodium (à 50% dans l'huile) dans 50 ml d'un mélange de tétrahydrofuranne/diméthylformamide (95/5), on ajoute une solution de 85 ml de tétrahydrofuranne/diméthylformamide (95/5) contenant 7 g (0,019 mole) d'amide obtenu selon 6.3. et 2,4 ml d'iodure de méthyle. On maintient la suspension sous agitation jusqu'à 1 heure après la fin de dégagement gazeux. On détruit l'excès d'hydrure de sodium par du méthanol puis on évapore les solvants sous pression réduite. On reprend le résidu solide à l'eau et au dichlorométhane. On décante la phase organique, on la sèche sur sulfate de sodium, on la filtre et on évapore le solvant. On purifie le solide obtenu sur colonne de chromatographie puis par recristallisation dans un mélange d'acétate d'éthyle et de cyclohexane.
On obtient 4,7 g (65%) de produit.
F = 157-159°C.

6,5. N-[[méthyl-6 (amino-4 phényl)-2 imidazo[1,2-a]pyridinyl-3]méthyl]-N,3-diméthyl-butanamide.

Dans 100 ml d'éthanol absolu, on met en réaction 3,5 g (0,0144 mole) d'amide obtenu selon 6.4., 3,42 g de fer en poudre et 6 g d'acide acétique. On chauffe la suspension à reflux pendant 4 heures 30 puis on la refroidit. On chasse l'éthanol et l'acide acétique sous pression réduite, on traite le résidu avec 350 ml d'eau et on extrait le produit au dichlorométhane. Après purification par chromatographie, on obtient 4,4 g (87%) de produit.
F = 178-179°C.

6.6. N-[[méthyl-6 [(pipéridinyl-1)-4 phényl]-2 imidazo[1,2-a] pyridinyl-3]méthyl]-N,3-diméthyl-butanamide.

Dans un ballon de 250 ml, on introduit successivement 1,7 g (0,00485 mole) d'amide obtenu selon 6.5.,

120 ml de toluène sec, 0,56 g (0,00243 mole) de dibromo-1,5 pentane et 0,75 g (0,0058 mole) d'éthyldiisopropylamine. On chauffe le mélange au reflux pendant 13 heures puis on le concentre sous pression réduite. On purifie directement le résidu huileux par chromatographie.
On obtient une huile jaunâtre dont on prépare le chlorhydrate dans un mélange d'alcool isopropylique et d'éther.
F = 116-118°C.

Exemple 7

N-[[méthyl-6 (diéthylamino-4 phényl)-2 imidazo[1,2-a]pyridinyl-3]méthyl]-N,3-diméthyl-butanamide.

Dans un ballon maintenu sous argon, on dissout 1,3 g (0,0037 mole) d'amide obtenu selon 6.5. dans 25 ml de méthanol contenant 0,6 g (0,0013 mole) d'acétaldéhyde. A ce mélange, on ajoute une solution de 0,5 g (0,0079 mole) de cyanoborohydrure de sodium et de 0,55 g (0,0039) mole de chlorure de zinc dans 8 ml de méthanol.
Après 1 heure de réaction, on ajoute 0,5 ml d'acide acétique et maintient l'agitation encore 2 heures à la température ambiante, puis une nuit à 50°C. On traite ensuite le mélange réactionnel avec 20 ml de soude 0,1 N et on chasse les solvants sous pression réduite. On traite le résidu d'évaporation avec du dichlorométhane, on lave la phase organique à l'eau, on la décante et on la sèche.
Après évaporation du solvant, on purifie le produit obtenu par chromatographie.
On obtient une huile dont on fait le dichlorhydrate dans un mélange d'alcool isopropylique et d'éther.
F = 158-160°C.

Exemple 8

N-[[(diméthyl-3,4 phényl)-2 méthyl-6 imidazo[1,2-a]pyridinyl-3]méthyl]-N,3-diméthyl-butanamide.

8.1. (Diméthyl-3,4 phényl)-2 méthyl-6 imidazo[1,2-a]pyridine.

A une solution de 42 g (0,188 mole) de (dimèthyl-3,4 phényl)-1 bromo-2 éthanone dans 400 ml d'éthanol à 95%, on ajoute 20,5 g (0,188 mole), d'amino-2 méthyl-5 pyridine et 32 g (0,381 mole) de bicarbonate de sodium. On chauffe la suspension à la température du reflux pendant 6 heures puis la refroidit et la concentre sous pression réduite. On lave le résidu à l'eau puis le traite par un mélange éther-éthanol et ensuite par un mélange éther-acétone et finalement par l'éther.
On obtient une poudre jaune d'une pureté suffisante pour continuer la synthèse.
F = 157-160°C.

8.2. (Diméthyl-3,4 phényl)-2 méthyl-6 imidazo[1,2-a]pyridine-3-méthanol.

On dissout 13,5 g (0,057 mole) de l'imidazopyridine obtenue précédemment dans 80 ml d'acide acétique, contenant 20 ml de formol à 37% dans l'eau. On porte la solution à 55°C pendant 4 heures puis la concentre sous pression réduite. On reprend le résidu d'évaporation à l'eau, puis le traite avec un excès d'ammoniaque jusqu'à pH basique. On agite la suspension formée 24 heures en présence de 200 ml de dichlorométhane puis on filtre l'insoluble sur fritté et le lave à l'eau puis ensuite à l'éther.
Après séchage sous vide on obtient un poudre blanche amorphe.
F : 209-210°C.

8.3. N-[[(diméthyl-3,4 phényl)-2 méthyl-6 imidazo[1,2-a]pyridinyl-3]méthyl]méthyl-3 butanamide

A une solution de 20 ml d'isovaléronitrile contenant 3,5 g (0,0131 mole) de d'alcool obtenu précédemment, on ajoute goutte à goutte, lentement, 3,5 ml d'acide sulfurique concentré. On maintient l'agitation pendant 1 heure 30 à la température ambiante, puis 2 heures à 110°C. On refroidit la suspension, la traite par 100 g de glace puis un excès d'ammoniaque jusqu'à pH basique.
On extrait l'insoluble au dichlorométhane et le purifie par chromatographie sur silice. On obtient l'amide secondaire (Ia) sous forme d'une huile visqueuse dont on prépare le chlorhydrate.
F = 217-218°C.

8.4. N-[[(diméthyl-3,4 phényl)-2 méthyl-6 imidazo[1,2-a]pyridinyl-3]méthyl]-N,3-diméthyl-butanamide

A une suspension de 1,16 g (0,0024 mole) d'hydrure de sodium (à 50% dans l'huile) dans 20 ml de tétrahydrofuranne contenant 1,26 ml, soit 2,87 g (0,002 mole), de iodométhane,on ajoute 3,53 g (0,001 mole) de l'amide secondaire obtenu précédemment, en solution dans 35 ml de tétrahydrofuranne. On maintient l'agitation pendant 3 heures. On traite la suspension par 1 ml de méthanol puis la concentre sous pression réduite. On reprend le résidu à l'eau, on extrait l'insoluble au dichlorométhane et le purifie par chromatographie sur silice.
F = 103-4°C
Le chlorhydrate du composé fond à 162,5-163,5°C.

Exemple 9

N-[[(diméthyl-3,4 phényl)-2 méthyl-6 imidazo[1,2-a]pyridinyl-3]méthyl]-N-méthyl-butanamide

9.1. N-[[(diméthyl-3,4 phényl)-2 méthyl-6 imidazo[1,2-a]pyridinyl-3]méthyl]-butanamide

On met en suspension 6 g (0,022 mole) de l'alcool obtenu selon 8.2. dans 50 ml de butyronitrile puis on ajoute lentement 6 ml d'acide sulfurique concentré. On agite le mélange à la température ambiante pendant 1 heure 30, puis on le porte 1/2 heure à 140°C. Après refroidissement, on traite à la glace la phase inférieure, la partie surnageante ayant été éliminée. Après dissolution complète de la gomme, on traite la solution avec un excès d'ammoniaque jusqu'à pH fortement basique. On extrait l'insoluble au dichlorométhane et le purifie par recristallisation dans un mélange cyclohexane/ acétate d'éthyle.
On obtient l'amide secondaire (Ia) dont on prépare le chlorhydrate.
F = 219-220°C.

9.2. N-[[(diméthyl-3,4 phényl)-2 méthyl-6 imidazo[1,2-a]pyridinyl-3]méthyl]-N-méthyl-butanamide

A une suspension de 1,19 g d'hydrure de sodium (à 50% dans de l'huile) dans 20 ml de tétrahydrofuranne sec contenant 1,29 ml soit 2,95 g (0,0208 mole) de iodométhane, on ajoute 3,49 g (0,0104 mole) de l'amide obtenu précédemment. On dilue le milieu réactionnel par 30 ml de tétrahydrofuranne. On maintient l'agitation pendant 2 heures puis après addition de 1 ml de méthanol, on concentre le mélange sous pression réduite. On traite le résidu à l'eau et extrait l'insoluble au dichlorométhane puis le chromatographie sur silice. On obtient une huile dont on prépare le chlorhydrate.
F = 181,5-182°C.

Exemple 10

N-[[diméthylamino-6 (méthyl-4 phényl)-2 imidazo[1,2-a]pyridinyl-3]méthyl]-N,3-diméthyl-butanamide.

10.1. N-[[diméthylamino-6 (méthyl-4 phényl)-2 imidazo[1,2-a] pyridinyl-3]méthyl]méthyl-3 butanamide.

Dans 83 ml de tétrahydrofuranne, on introduit 8 ml (0,0025 mole) d'acide sulfurique 3 M et 6 ml (0,072 mole) de formaldéhyde en solution aqueuse à 40 %. A cette solution on ajoute 4 g (0,0118 mole) d'amine préparée selon 3 et laisse agiter le mélange 20 mn. On refroidit au bain d'eau glacée et ajoute ensuite 3,12 g (0,0826 mole) de borohydrure de sodium par petites portions.
Le pH de la solution doit rester voisin de 4, au besoin on ajoute de l'acide sulfurique 3 M. En fin de réaction on traite le mélange par du carbonate de potassium en solution aqueuse et on extrait le produit au dichlorométhane. On lave et on sèche la phase organique et on évapore le solvant. On purifie le résidu d'évaporation par chromatographie sur colonne puis on le reprend à l'éther. On obtient 2,3 g (53%) de solide.
F = 173-175°C.

10.2. N-[[Diméthylamino-6 (méthyl-4 phényl)-2 imidazo[1,2-a] pyridinyl-3]méthyl]-N,3-diméthyl-butanamide.

On ajoute à une suspension de 0,526 g (0,0109 mole) d'hydrure de sodium à 50% dans l'huile dans un mélange de 20 ml de tétrahydrofuranne et 2 ml de diméthylformamide, 2 g (0,0054 mole) d'amide préparé selon 10.1 et 1,56 g (0,0109 mole) d'iodure de méthyle dissous dans un mélange de 25 ml de tétrahydrofuranne et 2,5 ml de diméthylformamide. Après la fin du dégagement gazeux, on maintient l'agitation pendant 1 heure

puis ajoute 1 ml de méthanol pour détruire l'excès d'hydrure de sodium. On évapore le solvant sous pression réduite, on reprend le résidu d'évaporation à l'eau et on extrait l'amide tertiaire au dichlorométhane. On purifie le produit par chromatographie flash sur colonne, puis on en fait le chlorhydrate que l'on recristallise dans la méthyléthylcétone. On obtient ainsi 0,6 g (27%) de sel.
F = 200-202°C.

Exemple 11

[[Méthyl(méthyl-3 butanoyl)amino]méthyl]-3 (méthyl-4 phényl)-2 imidazo[1,2-a]pyridinecarbonitrile-6.

Dans 100 ml de diméthylformamide, on introduit 3,7 g (0,041 mole) de cyanure cuivreux et 19 g (0,041 mole) de N-[[iodo-6 (méthyl-4 phényl)-2 imidazo[1,2-a]pyridinyl-3]méthyl]-N,3-diméthyl-butanamide préparé selon le procédé décrit dans la demande de brevet européen n° 0172096. On chauffe le mélange à reflux pendant 4 heures puis on évapore le solvant sous pression réduite. On reprend le résidu avec de l'eau et du dichlorométhane, et on traite le mélange avec de l'ammoniaque. On lave la phase organique à l'eau, on la sèche et on évapore le solvant. On purifie le résidu par chromatographie sur colonne et on le reprend avec du pentane. On obtient 14,4 g (96%) de solide blanc.
F = 151-153°C.

Exemple 12

[[Méthyl(méthyl-3 butanoyl)amino]méthyl]-3 (méthyl-4 phényl)-2 imidazo[1,2-a]pyridinecarboxamide-6.

Dans 36 ml d'acide formique, on introduit 3,6 g (0,01 mole) de nitrile préparé selon 11 puis on y fait passer un courant d'acide chlorhydrique gazeux sec à température ambiante jusqu'à transformation complète du nitrile. La réaction terminée, on verse la solution dans 200 ml d'eau, on ajoute de l'ammoniaque et on extrait l'amide avec du dichlorométhane. On le recristallise dans la méthyléthylcétone. On obtient 2,4 g (64%) d'amide.
F = 231-233°C.

Exemple 13

[[Méthyl(méthyl-3 butanoyl)amino]méthyl]-3 (méthyl-4 phényl)-2 imidazo[1,2-a]pyridinecarboxylate-6 d'éthyle.

Dans 36 ml d'éthanol sec, on introduit 3,6 g (0,01 mole) de nitrile préparé selon 11, puis on sature cette solution par de l'acide chlorhydrique gazeux en agitant plusieurs heures. Puis on traite la solution à l'eau pendant 5 heures, et on l'alcalinise avec du bicarbonate de sodium. On extrait l'ester avec du dichlorométhane et on le purifie par chromatographie sur colonne. On obtient 1,53 g (38%) de solide blanc.
F = 158-160°C.

Exemple 14

Acide [[méthyl(méthyl-3 butanoyl)amino]méthyl]-3 (méthyl-4 phényl)-2 imidazo[1,2-a]pyridinecarboxylique-6.

Dans un mélange de 35 ml d'acide acétique et 35 ml d'acide chlorhydrique concentré, on introduit 7,2 g (0,02 mole) de nitrile préparé selon 11. On chauffe cette solution à reflux plusieurs heures puis on la refroidit et on l'évapore à sec sous pression réduite. On reprend le résidu d'évaporation à l'eau, on l'amène à pH = 5 au moyen d'acide acétique. On sépare le précipité par filtration, on le lave à l'eau puis à l'acétone et on le sèche. On le recristallise dans le méthanol. On obtient 6,5 g (85%) d'acide.
F = 227-229°C.

Exemple 15

N-méthyl [[méthyl(méthyl-3 butanoyl)amino]méthyl]-3 (méthyl-4 phényl)-2 imidazo[1,2-a]pyridinecarboxamide-6.

Dans un ballon purgé à l'argon, on introduit 1,6 g (0,0042 mole) d'acide préparé selon 14, 0,82 g (0,005 mole) de carbonyldiimidazole et 20 ml de tétrahydrofuranne sec. On agite la solution 1 heure à 40°C puis on

la refroidit et la sature par un courant de méthylamine gazeuse sèche. On maintient l'agitation pendant une nuit puis on évapore le solvant. On reprend le résidu d'évaporation avec de l'eau et du dichlorométhane, on sépare la phase organique, on la lave à l'eau, on la sèche et on évapore le solvant. On purifie le résidu d'évaporation par chromatographie. On obtient 0,94 g (57%) d'amide.
F = 183-185°C.

Exemple 16

N,N-diméthyl [[méthyl(méthyl-3 butanoyl)amino]méthyl]-3 (méthyl-4 phényl)-2 imidazo[1,2-a]pyridinecarboxamide-6.

Dans un ballon purgé à l'argon, on introduit 1,6 g (0,0042 mole) d'acide préparé selon 14, 0,82 g (0,005 mole) de carbonydiimidazole et 20 ml de tétrahydrofuranne sec. On chauffe la suspension à 40°C pendant 2 h puis on le refroidit et on la sature avec un courant de diméthylamine gazeuse. En fin de réaction, on évapore la solution sous pression réduite, on reprend le résidu avec de l'eau et du dichlorométhane. On sépare la phase organique, on la sèche et on évapore le solvant. On purifie le résidu par chromatographie sur colonne. On obtient 0,89 g (52%) d'amide.
F = 134-136°C.

Exemple 17

[Méthyl-6 [(méthyl-3 butanoyl)aminométhyl]-3 imidazo[1,2-a] pyridinyl-2]-4 benzonitrile.

17.1 [Méthyl-6 imidazo[1,2-a]pyridinyl-2]-4 benzonitrile.

Dans 1,2 1 d'éthanol à 95% on introduit 70 g (0,647 mole) de méthyl-5 pyridineamine-2, 145 g (0,647 mole) de (bromo-2 acétyl)-4 benzonitrile et 110 g (1,294 mole) de bicarbonate de soude. On chauffe la suspension à reflux pendant 2 h puis on la filtre et on l'évapore à sec. On reprend le résidu à l'eau et on le traite au dichlorométhane. On sépare la phase organique, on la sèche et on évapore le solvant. On reprend le résidu à l'éther. On obtient 107 g (71%) d'imidazopyridine.
F = 228-230°C.

17.2. [Méthyl-6 hydroxyméthyl-3 imidazo[1,2-a]pyridinyl-2]-4 benzonitrile.

Dans un ballon tricol de 21, on introduit 70 g (0,3 mole) d'imidazopyridine préparé selon 17.1, 1,5 l d'acide acétique et 210 ml de formaldéhyde en solution à 37% dans l'eau. On agite le mélange pendant 3 h à 50°C puis on évapore l'acide acétique et l'eau. On reprend le résidu avec de l'eau et du dichlorométhane, puis on le traite par un excès d'ammoniaque et l'alcool précipite. On le filtre, le lave à l'acétone et le sèche sous vide. On obtient 52 g (66%) d'alcool.

17.3. [Méthyl-6 [(méthyl-3 butanoyl)aminométhyl]-3 imidazo [1,2-a]pyridinyl-2]-4 benzonitrile.

Dans un ballon de 250 ml on introduit 500 ml d'isovaléronitrile, 50 g (0,189 mole) d'alcool préparé selon 17.2, puis on ajoute rapidement à ce mélange 57 ml d'acide sulfurique concentré. On chauffe la suspension jusqu'à obtention de deux phases liquides. On hydrolyse le mélange biphasique à la glace puis on le traite par un excès d'ammoniaque. On filtre le précipité formé, le lave à l'eau et le sèche sous vide. On le purifie par chromatographie sur colonne. On obtient 24 g (37%) de produit.
F = 231-233°C.

Le tableau suivant illustre les structures et propriétés physiques de quelques composés selon l'invention.

Tableau

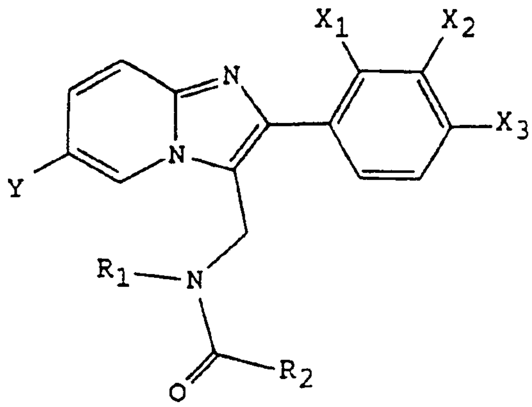

(I)

| Composé | Y | $X_1$ | $X_2$ | $X_3$ | $R_1$ | $R_2$ | F(°C) |
|---|---|---|---|---|---|---|---|
| 1 (Ex. 1.3) | $CF_3$ | H | H | $CH_3$ | H | $iC_4H_9$ | 212-213 |
| 2 (Ex. 1.4) | $CF_3$ | H | H | $CH_3$ | $CH_3$ | $iC_4H_9$ | 196-197 |
| 3 | $CF_3$ | H | H | $SCH_3$ | H | $iC_4H_9$ | 231-232 |
| 4 | $CF_3$ | H | H | $SCH_3$ | $CH_3$ | $iC_4H_9$ | 180-182 |
| 5 (Ex. 2.3) | $NO_2$ | H | H | $CH_3$ | H | $iC_4H_9$ | 239-241 |
| 6 (Ex. 3) | $NH_2$ | H | H | $CH_3$ | H | $iC_4H_9$ | 245-247 |
| 7 (Ex. 4.3) | $CH_3$ | H | H | $CF_3$ | H | $iC_4H_9$ | 200-202 |
| 8 (Ex. 4.4) | $CH_3$ | H | H | $CF_3$ | $CH_3$ | $iC_4H_9$ | 129-130 |
| 9 (Ex. 5.3) | $CH_3$ | H | H | $CO_2C_2H_5$ | H | $iC_4H_9$ | 230-231 |
| 10 (Ex. 6.6) | $CH_3$ | H | H | 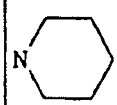 | $CH_3$ | $iC_4H_9$ | 116-118 |
| 11 | $CH_3$ | H | H | 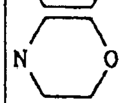 | $CH_3$ | $iC_4H_9$ | 137,5-138 |
| 12 (Ex. 7) | $CH_3$ | H | H | $N(C_2H_5)_2$ | $CH_3$ | $iC_4H_9$ | 158-160** |
| 13 | $CH_3$ | H | H | $NHC_2H_5$ | $CH_3$ | $iC_4H_9$ | 173-174 |
| 14 | $CH_3$ | H | H | 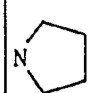 | $CH_3$ | $iC_4H_9$ | 153-154** |
| 15 | $NHCH_3$ | H | H | $CH_3$ | H | $iC_4H_9$ | 252-254 |
| 16 (Ex. 10.1) | $N(CH_3)_2$ | H | H | $CH_3$ | H | $iC_4H_9$ | 173-175 |
| 17 | $CH_3$ | Cl | H | Cl | $CH_3$ | $nC_3H_7$ | 146-147* |
| 18 | $CH_3$ | H | Cl | Cl | $CH_3$ | $nC_3H_7$ | 124-125 |

Tableau (suite)

| Composé | Y | $X_1$ | $X_2$ | $X_3$ | $R_1$ | $R_2$ | F(°C) |
|---|---|---|---|---|---|---|---|
| 19 | $CH_3$ | H | Cl | H | $CH_3$ | $nC_3H_7$ | 84-85 |
| 20 | $CH_3$ | Cl | H | H | $CH_3$ | $nC_3H_7$ | 185-186 |
| 21 | $CH_3$ | H | $CH_3$ | H | $CH_3$ | $nC_3H_7$ | 94,5-95 |
| 22 | $CH_3$ | H | $OCH_3$ | H | $CH_3$ | $nC_3H_7$ | 165-166* |
| 23 | $CH_3$ | $CH_3$ | H | $CH_3$ | $CH_3$ | $nC_3H_7$ | 129-129,5 |
| 24 (Ex. 9.2) | $CH_3$ | H | $CH_3$ | $CH_3$ | $CH_3$ | $nC_3H_7$ | 181,5-182* |
| 25 | $CH_3$ | $CH_3$ | H | $CH_3$ | $CH_3$ | $iC_4H_9$ | 111-112* |
| 26 (Ex. 8.4) | $CH_3$ | H | $CH_3$ | $CH_3$ | $CH_3$ | $iC_4H_9$ | 162,5-163,5* |
| 27 | $CH_3$ | H | $OCH_3$ | $OCH_3$ | $CH_3$ | $nC_3H_7$ | 168-168,5* |
| 28 | Cl | H | $CH_3$ | $CH_3$ | $CH_3$ | $nC_3H_7$ | 124,5-125 |
| 29 | $CH_3$ | Cl | H | Cl | H | $nC_3H_7$ | 160-161 |
| 30 | $CH_3$ | H | Cl | Cl | H | $nC_3H_7$ | 160-161 |
| 31 | $CH_3$ | H | Cl | H | H | $nC_3H_7$ | 154-155 |
| 32 | $CH_3$ | Cl | H | H | H | $nC_3H_7$ | 178-179 |
| 33 | $CH_3$ | H | $CH_3$ | H | H | $nC_3H_7$ | 141-142 |
| 34 | $CH_3$ | H | $OCH_3$ | H | H | $nC_3H_7$ | 187,5-188,5* |
| 35 | $CH_3$ | $CH_3$ | H | $CH_3$ | H | $nC_3H_7$ | 160-161 |
| 36 (Ex. 9.1) | $CH_3$ | H | $CH_3$ | $CH_3$ | H | $nC_3H_7$ | 219-220* |
| 37 | $CH_3$ | $CH_3$ | H | $CH_3$ | H | $iC_4H_9$ | 166-167 |
| 38 (Ex. 8.3) | $CH_3$ | H | $CH_3$ | $CH_3$ | H | $iC_4H_9$ | 217-218* |
| 39 | $CH_3$ | H | $OCH_3$ | $OCH_3$ | H | $nC_3H_7$ | 200-201* |
| 40 | $NHCH_3$ | H | H | $CH_3$ | $CH_3$ | $iC_4H_9$ | 175-177 |
| 41 (Ex. 10.2) | $N(CH_3)_2$ | H | H | $CH_3$ | $CH_3$ | $iC_4H_9$ | 200-202* |
| 42 (Ex. 11) | CN | H | H | $CH_3$ | $CH_3$ | $iC_4H_9$ | 151-153 |
| 43 (Ex. 12) | $CONH_2$ | H | H | $CH_3$ | $CH_3$ | $iC_4H_9$ | 231-233 |
| 44 (Ex. 13) | $CO_2C_2H_5$ | H | H | $CH_3$ | $CH_3$ | $iC_4H_9$ | 158-160 |
| 45 (Ex. 15) | $CONHCH_3$ | H | H | $CH_3$ | $CH_3$ | $iC_4H_9$ | 183-185 |
| 46 (Ex. 14) | $CO_2H$ | H | H | $CH_3$ | $CH_3$ | $iC_4H_9$ | 227-229 |
| 47 (Ex. 16) | $CON(CH_3)_2$ | H | H | $CH_3$ | $CH_3$ | $iC_4H_9$ | 134-136 |
| 48 (Ex. 17.3) | $CH_3$ | H | H | CN | H | $iC_4H_9$ | 231-233 |
| 49 | $CH_3$ | H | H | CN | $CH_3$ | $iC_4H_9$ | 147-149 |
| 50 | $CH_3$ | H | H | $CONH_2$ | H | $iC_4H_9$ | 271-272 |

Tableau (suite)

| Composé | Y | $X_1$ | $X_2$ | $X_3$ | $R_1$ | $R_2$ | F(°C) |
|---|---|---|---|---|---|---|---|
| 51 | $CH_3$ | H | H | $CONH_2$ | $CH_3$ | $iC_4H_9$ | 225-227 |
| 52 | $CH_3$ | H | H | $CO_2C_2H_5$ | $CH_3$ | $iC_4H_9$ | 113-115 |
| 53 | $CH_3$ | H | H | $CONHCH_3$ | $CH_3$ | $iC_4H_9$ | 189-190 |
| 54 | $CH_3$ | H | H | $CON(CH_3)_2$ | $CH_3$ | $iC_4H_9$ | 116-118 |
| 55 | $CH_3$ | H | H | $CO_2H$ | $CH_3$ | $iC_4H_9$ | 240-242 |

* = chlorhydrate.

** = dichlorhydrate

Les composés de l'invention ont fait l'objet d'essais pharmacologiques qui ont misq en évidence leur intérêt comme substances à activités thérapeutiques.

Toxicité aiguë.

Elle a été déterminée chez la souris par voie intrapéritonéale. Les DL 50 sont supérieures à 500 mg/kg.

Antagonisme vis-à-vis des convulsions cloniques induites par le Cardiazol® chez la souris.

L'essai est inspiré du protocole décrit par Goodman et al., J. Pharm. Exp. Ther., (1953), 108, 168-176. Les souris reçoivent les produits à tester, ou le solvant seul, par voie intrapéritonéale 30 minutes avant l'injection de 35 mg/kg de Cardiazol® par voie intraveineuse. Les animaux sont ensuite observés pendant une heure et, pour chaque lot, le pourcentage de souris présentant des convulsions cloniques est noté (100% de convulsions cloniques et 10 a 20% de convulsions toniques chez les animaux de contrôle).
Pour chaque dose, on calcule le pourcentage de protection par rapport aux animaux de contrôle, ce qui permet de déterminer graphiquement la DA50, dose qui protège 50% des animaux vis-à-vis des effets convulsivants du Cardiazol®.
Les DA50 des composés de l'invention se situent entre 0,1 et plus de 30 mg/kg.

Action sur l'électrocorticogramme du rat curarisé ventilé.

L'activité sédative ou hypnotique des composés a été déterminée par l'observation de leur action sur l'électrocorticogramme du rat selon la méthode décrite par H. Depoortere, Rev. E.E.G. Neurophysiol., 10, 3, 207-214 (1980) et par H. Depoortere et M. Decobert, J. Pharmacol. (Paris), 14, 2, 195-265 (1983).
Les produits à étudier ont été administrés par voie intrapéritonéale aux, :doses croissantes de 1 à 30 mg/kg. Ils induisent dés tracés de sommeil à partir de doses allant de 0,01 à 30 mg/kg.

Effets sur la durée du "sommeil" induit par le hydroxy-4 butyrate de sodium.

Cette action a été déterminée par l'influence d'un composé sur la durée du "sommeil" induit par le hydroxy-4 butyrate de sodium (GHB) chez le rat curarisé.

Les animaux utilisés sont des rats mâles de souche Charles River de 200 ± 20 g. Les animaux, curarisés par l'alloférine à raison de 1 mg/kg par voie i.p., sont placés sous respiration artificielle à l'aide d'un masque appliqué sur le museau (fréquence respiratoire = 50/minute ; volume respiratoire = 14 ml).
L'oesophage est préalablement ligaturé afin d'éviter l'entrée de l'air dans l'estomac.

Des électrodes corticales front-pariétales et occipitales permettent l'enregistrement de l'activité électrocor-

ticographique sur un polygraphe Grass 79 P à la vitesse de 6 mm/s. La préparation de l'animal est effectuée sous anesthésie locale (xylocaïne à 2%). Les rats sont maintenus tout au long de l'expérience à température constante (37,5°C). Dix minutes après la fin de la préparation du rat, une dose de 200 mg/kg de hydroxy-4 butyrate de sodium est injectée par voie intraveineuse au niveau de la queue.
Une dose de 10 mg/kg du composé à étudier est administrée par voie intrapéritonéale 3 minutes après l'administration du hydroxy-4 butyrate de sodium.

L'évaluation des tracés s'effectue par périodes de 15 minutes durant 75 minutes après l'injection de GHB. Durant cette période d'analyse, la durée totale du "sommeil" est déterminée. Une série de 15 témoins permet de préciser la durée du "sommeil1 GHB".
L'analyse statistique des résultats est réalisée à l'aide du test "U" de Mann-Whitney.
Certains composés réduisent les effets du GHB (jusqu'à 40% de diminution de la durée du sommeil à la dose de 10 mg/kg), tandis que d'autres potentialisent ces effets (jusqu'à 25%d'augmentation de la durée du sommeil à la dose de 10 mg/kg). On constate également que les effets peuvent être opposés selon que les composés sont administrés à doses fortes ou faibles.

Test de conflit de la boisson chez le rat.

Ce test est décrit par Vogel J.R., Beer B. et Clody D.E. dans Psychopharmacologia, 21, 1-7, (1971).

Des rats mâles Wistar (IFFA Credo) sont utilisés. L'eau de boisson leur est retirée 24 h avant le test. Le jour du test, 30 minutes après traitement par voie intrapéritonéale avec les composés de l'invention, chaque rat est placé dans une cage en matière plastique transparente (24 × 20 × 21 cm) à plancher grillagé électrifiable. De l'eau de boisson est distribuée par l'intermédiaire d'une pipette sortant de 2 cm d'une paroi de la cage et placée à 3 cm au-dessus du plancher de la cage.
Après une exploration de 10 à 90 secondes, l'animal trouve la pipette et commence à boire. Après avoir donné 20 coups de langue (enregistrés par un anxiomètre OMNITECH), le rat reçoit, au niveau de la langue un choc électrique de 0,07 mA (délivré par l'anxiomètre) qui s'arrête lorsque le rat quitte la pipette. Une session de 3 minutes commence après un premier choc, l'animal continuant de recevoir un choc tous les 20 coups de langue jusqu'à ce qu'il s'arrête ou jusqu'à la fin de la session.
Dans ces conditions expérimentales, les animaux de contrôle acceptent, en moyenne, 3 à 6 chocs. Le nombre de chocs obtenus avec les animaux traités est noté, et on compare ce nombre avec celui des animaux témoins par un test de Dunett. On détermine ainsi la DEM, dose efficace minimale, qui est la première dose augmentant de façon significative le nombre de chocs acceptés par un animal, par rapport aux animaux témoins.
Les DEM des composés se situent entre 1 et 100 mg/kg par voie intrapéritonéale.

Activité analgésique.

Elle a été montrée dans le test de Koster et al., ("writhing test" à l'acide acétique chez la souris), Fed. Proc., 18, 412, 1959.
On administre par voie orale, aux souris à jeun, le composé à tester en solution dans du Tween 80® à 1%, à raison de 0,2 ml par 20 g de poids corporel ; au bout de 30 mn on administre l'acide acétique (en solution à 0,6% dans un mélange carboxyméthyl-cellulose et Tween 80®, à raison de 10 ml par kg de poids corporel) par voie intrapéritonéale. On note le nombre total de contorsions pendant 15 mn.
On détermine le pourcentage de protection par rapport à un lot témoin et on calcule la DA 50 par voie graphique (dose qui protège 50% des animaux).
La DA 50 des composés de l'invention va de 5 à 50 mg/kg p.o.

Ulcère de stress

La technique utilisée est celle de Senay et Levine, Proc. Soc. Exp. Biol. 1967, 124, 1221-1223, Peptic Ulcers, sur des rats Wistar femelles pesant 180-210 g, tenus à jeun depuis 20 heures, répartis en blocs randomisés.
Les animaux sont mis en contention dans des boîtes cylindriques de 20 cm × 5 cm et placés dans une chambre froide dont la température est maintenue entre 2 et 4°C.
Les composés à étudier sont administrés par voie orale à raison de 10, 30 et 100 mg/kg immédiatement avant la mise en contention, les rats témoins recevant seulement le placébo. 2 heures plus tard, les animaux sont sacrifiés par inhalation de chloroforme.
Les estomacs sont prélevés et le degré d'ulcération est noté. Les composés de l'invention diminuent significativement les ulcères de stress à des doses allant de 0,1 à 10 mg/kg par la voie orale.

Les résultats de ces différents tests montrent que les composés de l'invention possèdent des propriétés anxiolytiques, inductrices de sommeil, hypnotiques, anticonvulsivantes, analgésiques, et anti-ulcères de stress. Les composés de l'invention sont utiles pour le traitement des états d'anxiété, des troubles du sommeil et autres affections neurologiques et psychiatriques, pour le traitement des troubles de la vigilance, en particulier pour luttre contre les troubles du comportement imputables à des dommages vasculaires cérébraux et à la sclérose cérébrale en gériatrie, pour le traitement des absences dues à des traumatismes crâniens et pour le traitement des encéphalopathies métaboliques ainsi que pour le traitement de la douleur, des algies diverses et des ulcères de stress.

Les composés de l'invention peuvent être présentés sous toute forme appropriée pour l'administration par voie orale, ou parentérale, par exemple sous forme de comprimés, de dragées, de gélules, de solutions buvables ou injectables etc, en association avec tout excipient approprié.

La posologie quotidienne peut aller de 0,1 à 500 mg.

## Revendications

### Revendications pour les Etats Contractants : BE, CH, DE, FR, GB, IT, LI, LU, NL, SE

1. Composés de formule générale (I)

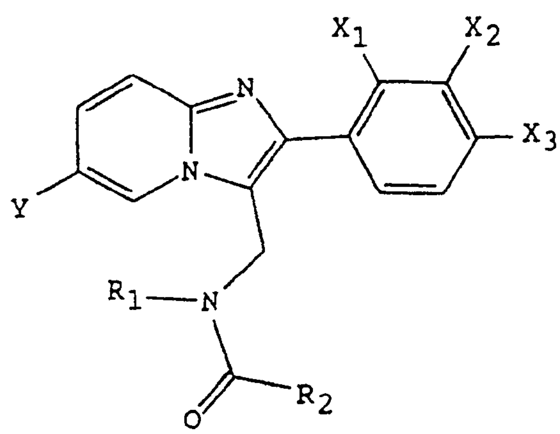

(I)

dans laquelle

$R_1$ représente un atome d'hydrogène ou un groupe alkyle en $C_1$-$C_4$ linéaire ou ramifié, ou un groupe benzyle, $R_2$ représente un groupe alkyle en $C_1$-$C_6$ linéaire ou ramifié, un groupe cyclohexyle, trichlorométhyle, propène-1 yle, allyle, phényle, chloro-4 phényle ou benzyle, ou bien encore

$R_1$ et $R_2$ représentent ensemble une chaîne aliphatique en $C_3$-$C_5$,

et, ou bien,

a) $X_1$ et $X_2$ représentent chacun un atome d'hydrogène, $X_3$ représente un atome d'halogène ou un groupe alkyle en $C_1$-$C_3$, un groupe méthylthio, un groupe trifluorométhyle, un groupe carboxy éventuellement estérifié (de formule COOR dans laquelle R représente l'hydrogène ou un alkyle en $C_1$-$C_6$), un groupe cyano, un groupe aminocarbonyle éventuellement mono-ou di-alkylé (de formule $CONR_3R_4$ dans laquelle $R_3$ et $R_4$, indépendamment l'un de l'autre, représentent chacun l'hydrogène ou un alkyle en $C_1$-$C_4$ ou représentent ensemble une chaîne de formule $(CH_2)_2$-Z-$(CH_2)_2$ dans laquelle Z représente une liaison ou un atome d'oxygène ou de soufre ou un groupe de formule $CH_2$, NH ou N(alkyle en $C_1$-$C_4$)), un groupe alkylamino (de formule $NHR_5$ dans laquelle $R_5$ représente un alkyle en $C_2$-$C_6$) ou dialkylamino (de formule $NR_6R_7$ dans laquelle $R_6$ et $R_7$, indépendamment l'un de l'autre, représentent chacun un alkyle en $C_2$-$C_6$ ou représentent ensemble une chaîne de formule $(CH_2)_2$-Z-$(CH_2)_2$ dans laquelle Z représente une liaison ou un atome d'oxygène ou de soufre ou un groupe de formule $CH_2$, NH ou N(alkyle en $C_1$-$C_4$)), et

Y représente un groupe méthyle, un groupe trifluorométhyle, u groupe carboxy éventuellement estérifié (de formule $COOR_8$ dans laquelle $R_8$ représente l'hydrogène ou un alkyle en $C_1$-$C_6$), un groupe cyano, un groupe aminocarbonyle éventuellement mono- ou di-alkylé (de formule $CONR_9R_{10}$ dans laquelle $R_9$ et $R_{10}$, indépendamment l'un de l'autre, représentent chacun l'hydrogène ou un alkyle en $C_1$-$C_4$), ou un groupe amino éventuellement mono- ou dialkylé (de formule $NR_{11}R_{12}$ dans laquelle $R_{11}$ et $R_{12}$, indépendamment l'un de l'autre, représentent chacun l'hydrogène ou un alkyle en $C_1$-$C_4$),

étant exclus dans le cas (a) les composés de formule (I) dans laquelle $X_3$ représente un atome d'halogène ou un groupe alkyle ou méthylthio et Y représente un groupe méthyle, ou bien

b) $X_1$, $X_2$ et $X_3$ représentent chacun, indépendamment l'un de l'autre, l'hydrogène, un halogène ou un groupe alkyle en $C_1$-$C_4$, méthoxy, méthylthio, éthylthio, méthylsulfonyle, nitro, amino, méthylamino, diméthylamino, acétylamino ou diacétylamino, et
Y représente un groupe méthyle,
étant exclus dans le cas (b) les composés dans la formule (I) desquels $X_1$ et $X_2$ représentent chacun un atome d'hydrogène, ainsi que leurs sels d'addition à des acides acceptables en pharmacologie.

2. Composés selon la revendication 1, caractérisés en ce que, dans la formule (I), $R_1$ représente un atome d'hydrogène ou un groupe méthyle, et $R_2$ représente un groupe propyle ou isobutyle.

3. Procédé de préparation de composés selon la revendication 1, caractérisé en ce qu'on fait réagir une amino-2 pyridine de formule (II)

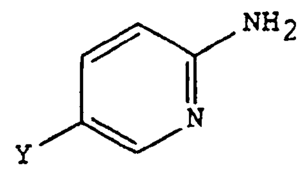

(II)

(dans laquelle Y est tel que défini ci-dessus), avec une bromo-2 éthanone de formule (III)

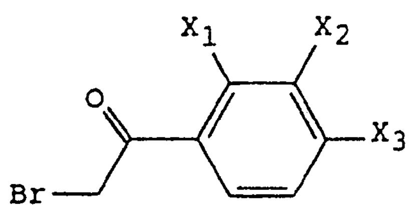

(III)

(dans laquelle $X_1$, $X_2$ et $X_3$ sont tels que définis ci-dessus), à chaud et dans un solvant protique,
puis on soumet l'imidazo(1,2-a)pyridine de formule (IV) ainsi obtenue

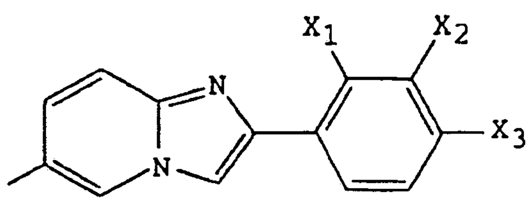

(IV)

à une hydroxyméthylation au moyen de formaldéhyde dans un solvant de type acide carboxylique,
puis on fait réagir l'alcool de formule (V) ainsi obtenu

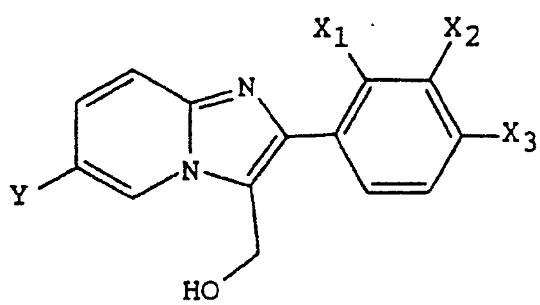

(V)

avec un nitrile de formule $R_2$-CN (dans laquelle $R_2$ est tel que défini ci-dessus), en milieu acide et à une température de 0 à 150°C,
puis, après hydrolyse du produit de la réaction, et si on le désire, on soumet le composé de formule (Ia) ainsi obtenu,

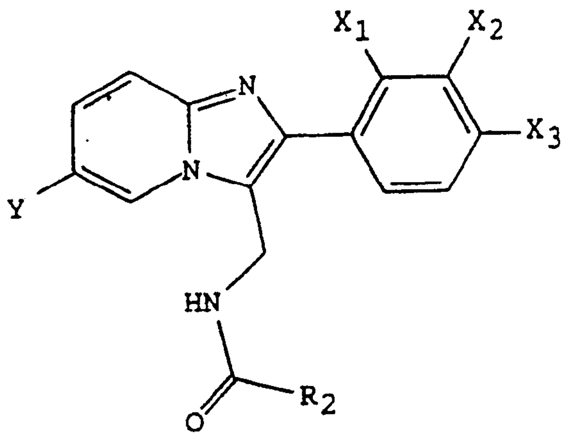

(Ia)

à une alkylation ou une benzylation par un halogénure de formule $R_1$-hal (dans laquelle $R_1$ est tel que défini ci-dessus), dans un solvant et en présence d'un hydrure de métal alcalin.

4. Procédé selon la revendication 3, caractérisé en ce que, après la réaction entre les composés de formules (II) et (III), on fait réagir l'imidazo[1,2-a]pyridine de formule (IV) ainsi obtenue avec un amide de formule

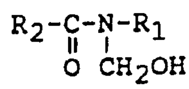

(dans laquelle $R_1$ et $R_2$ sont tels que définis ci-dessus), à température ambiante et en présence d'acide sulfurique concentré.

5. Procédé selon la revendication 3, caractérisé en ce que, après la réaction entre les composés de formules (II) et (III), on prépare un composé de formule (Ia) en faisant réagir l'imidazo[1,2-a]pyridine de formule (IV) avec un nitrile de formule $R_2$-CN et avec le paraformaldéhyde, à chaud, en milieu acétique et en présence d'acide sulfurique.

6. Procédé selon la revendication 3, caractérisé en ce que, pour obtenir un composé de formule (I) dans laquelle $X_3$ et/ou Y représentent un groupe cyano, on fait réagir un composé de formule (I), dans laquelle $X_3$ et/ou Y représentent un atome d'iode, avec un cyanure métallique dans un solvant inerte.

7. Procédé selon la revendication 3, caractérisé en ce que, pour obtenir un composé de formule (I) dans laquelle $X_3$ et/ou Y représentent un groupe carboxylate, on fait réagir un composé de formule (I), dans laquelle $X_3$ et/ou Y représentent un groupe cyano, avec l'acide chlorhydrique gazeux dans un alcool de formule ROH ou $R_8$OH, R et $R_8$ étant tels que définis ci-dessus.

8. Procédé selon la revendication 3, caractérisé en ce que, pour obtenir un composé de formule (I) dans laquelle $X_3$ et/ou Y représentent un groupe carboxyle ou carboxamide on hydrolyse un composé de formule (I), dans laquelle $X_3$ et/ou Y représentent un groupe cyano, en milieu acide, à une température de 60 à 120°C.

9. Procédé selon la revendication 3, caractérisé en ce que, pour obtenir un composé de formule (I) dans laquelle $X_3$ et/ou Y représentent un groupe carboxamide, on amidifie un composé de formule (I), dans laquelle $X_3$ et/ou Y représentent un groupe carboxyle, au moyen de carbonyldiimidazole, puis on traite l'imidazolide intermédiaire ainsi obtenu in situ par un amide de formules $R_3R_4NH$ ou $R_9R_{10}$, respectivement, dans lesquelles $R_3$, $R_4$, $R_9$ et $R_{10}$ sont tels que définis ci-dessus.

10. Médicament, caractérisé en ce qu'il consiste en un composé selon l'une des revendications 1 et 2.

11. Composition pharmaceutique, caractérisée en ce qu'elle contient un composé selon l'une des revendications 1 et 2, associé à un excipient approprié.

**Revendications pour les Etats Contractants : AT, ES, GR**

1. Procédé de préparation de composés de formule générale (I)

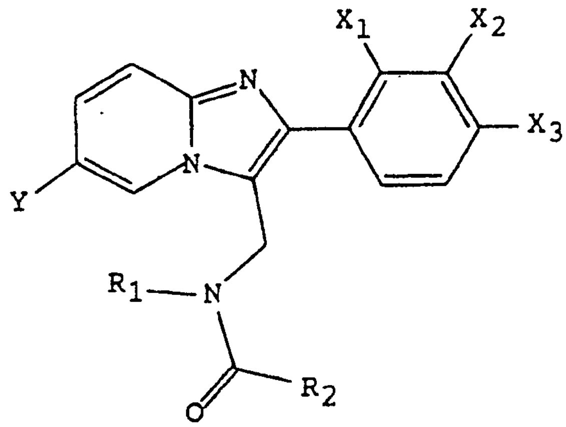

(I)

dans laquelle

$R_1$ représente un atome d'hydrogène ou un groupe alkyle en $C_1$-$C_4$ linéaire ou ramifié, ou un groupe benzyle,

$R_2$ représente un groupe alkyle en $C_1$-$C_6$ linéaire ou ramifié, un groupe cyclohexyle, trichlorométhyle, propène-1 yle, allyle, phényle, chloro-4 phényle ou benzyle, ou bien encore

$R_1$ et $R_2$ représentent ensemble une chaîne aliphatique en $C_3$-$C_5$,

et, ou bien,

a) $X_1$ et $X_2$ représentent chacun un atome d'hydrogène, $X_3$ représente un atome d'halogène ou un groupe alkyle en $C_1$-$C_3$, un groupe méthylthio, un groupe trifluorométhyle, un groupe carboxy éventuellement estérifié (de formule COOR dans laquelle R représente l'hydrogène ou un alkyle en $C_1$-$C_6$), un groupe cyano, un groupe aminocarbonyle éventuellement mono- ou di-alkylé (de formule $CONR_3R_4$ dans laquelle $R_3$ et $R_4$, indépendamment l'un de l'autre, représentent chacun l'hydrogène ou un alkyle en $C_1$-$C_4$ ou représentent ensemble une chaîne de formule $(CH_2)_2$-Z-$(CH_2)_2$ dans laquelle Z représente une liaison ou un atome d'oxygène ou de soufre ou un groupe de formule $CH_2$, NH ou N(alkyle en $C_1$-$C_4$)), un groupe alkylamino (de formule $NHR_5$ dans laquelle $R_5$ représente un alkyle en $C_2$-$C_6$) ou dialkylamino (de formule $NR_6R_7$ dans laquelle $R_6$ et $R_7$, indépendamment l'un de l'autre, représentent chacun un alkyle en $C_2$-$C_6$ ou représentent ensemble une chaîne de formule mule $(CH_2)_2$-Z-$(CH_2)_2$ dans laquelle Z représente une liaison ou un atome d'oxygène ou de soufre ou un groupe de formule $CH_2$, NH ou N(alkyle en $C_1$-$C_4$)), et

Y représente un groupe méthyle, un groupe trifluorométhyle, un groupe carboxy éventuellement estérifié (de formule $COOR_8$ dans laquelle $R_8$ représente l'hydrogène ou un alkyle en $C_1$-$C_6$), un groupe cyano, un groupe aminocarbonyle éventuellement mono- ou di-alkylé (de formule $CONR_9R_{10}$ dans laquelle $R_9$ et $R_{10}$, indépendamment l'un de l'autre, représentent chacun l'hydrogène ou un alkyle en $C_1$-$C_4$), ou un groupe amino éventuellement mono- ou dialkylé (de formule $NR_{11}R_{12}$ dans laquelle $R_{11}$ et $R_{12}$, indépendamment l'un de l'autre, représentent chacun l'hydrogène ou un alkyle en $C_1$-$C_4$),

étant exclus dans le cas (a) les composés de formule (I) dans laquelle $X_3$ représente un atome d'halogène ou un groupe alkyle ou méthylthio et Y représente un groupe méthyle,

ou bien

b) $X_1$, $X_2$ et $X_3$ représentent chacun, indépendamment l'un de l'autre, l'hydrogène, un halogène ou un groupe alkyle en $C_1$-$C_4$, méthoxy, méthylthio, éthylthio, méthylsulfonyle, nitro, amino, méthylamino, diméthylamino, acétylamino ou diacétylamino, et

Y représente un groupe méthyle,

étant exclus dans le cas (b) les composés dans la formule (I) desquels $X_1$ et $X_2$ représentent chacun un atome d'hydrogène, procédé caractérisé en ce qu'on fait réagir une amino-2 pyridine de formule (II)

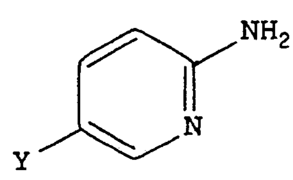

(II)

(dans laquelle Y est tel que défini ci-dessus), avec une bromo-2 éthanone de formule (III)

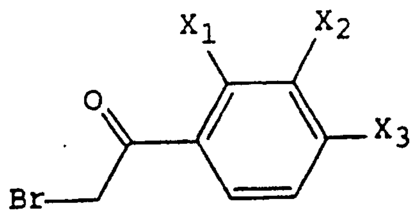

(III)

(dans laquelle $X_1$, $X_2$ et $X_3$ sont tels que définis ci-dessus), à chaud et dans un solvant protique, puis on soumet l'imidazo[1,2-a]pyridine de formule (IV) ainsi obtenue

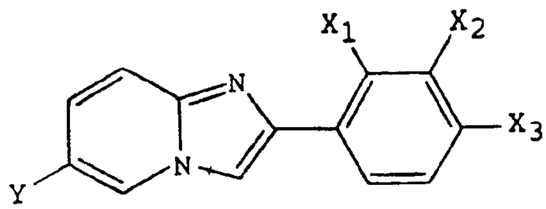

(IV)

à une hydroxyméthylation au moyen de formaldéhyde dans un solvant de type acide carboxylique, puis on fait réagir l'alcool de formule (V) ainsi obtenu

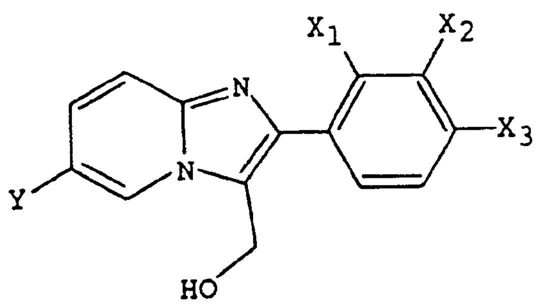

(V)

avec un nitrile de formule $R_2$-CN (dans laquelle $R_2$ est tel que défini ci-dessus), en milieu acide et à une température de 0 à 150°C,
puis, après hydrolyse du produit de la réaction, et si on le désire, on soumet le composé de formule (Ia) ainsi obtenu,

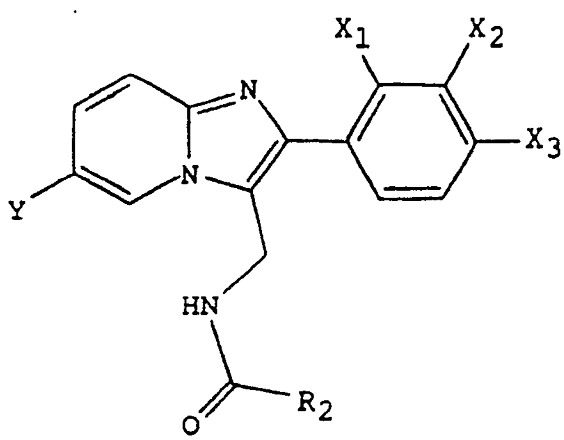

(Ia)

à une alkylation ou une benzylation par un halogénure de formule $R_1$-hal (dans laquelle $R_1$ est tel que défini ci-dessus), dans un solvant et en présence d'un hydrure de métal alcalin.

2. Procédé selon la revendication 1, caractérisé en ce que, après la réaction entre les composés de formules (II) et (III), on fait réagir l'imidazo(1,2-a]pyridine de formule (IV) ainsi obtenue avec un amide de formule

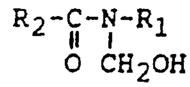

(dans laquelle $R_1$ et $R_2$ sont tels que définis ci-dessus), à température ambiante et en présence d'acide sulfurique concentré.

3. Procédé selon la revendication 1, caractérisé en ce que, après la réaction entre les composés de formules (II) et (III), on prépare un composé de formule (Ia) en faisant réagir l'imidazo(1,2-a]pyridine de formule (IV) avec un nitrile de formule $R_2$-CN et avec le paraformaldéhyde, à chaud, en milieu acétique et en présence d'acide sulfurique.

4. Procédé selon la revendication 1, caractérisé en ce que, pour obtenir un composé de formule (I) dans laquelle $X_3$ et/ou Y représentent un groupe cyano, on fait réagir un composé de formule (I), dans laquelle $X_3$ et/ou Y représentent un atome d'iode, avec un cyanure métallique dans un solvant inerte.

5. Procédé selon la revendication 1, caractérisé en ce que, pour obtenir un composé de formule (I) dans laquelle $X_3$ et/ou pour obtenir un composé de formule (I) dans laquelle $X_3$ et/ou Y représentent un groupe carboxylate, on fait réagir un composé de formule (I), dans laquelle $X_3$ et/ou Y représentent un groupe cyano, avec l'acide chlorhydrique gazeux dans un alcool de formule ROH ou $R_8$OH, R et $R_8$ étant tels que définis ci-dessus.

6. Procédé selon la revendication 1, caractérisé en ce que, pour obtenir un composé de formule (I) dans laquelle $X_3$ et/ou Y représentent un groupe carboxyle ou carboxamide on hydrolyse un composé de formule (I), dans laquelle $X_3$ et/ou Y représentent un groupe cyano, en milieu acide, à une température de 60 à 120°C.

7. Procédé selon la revendication 1, caractérisé en ce que, pour obtenir un composé de formule (I) dans laquelle $X_3$ et/ou Y représentent un groupe carboxamide, on amidifie un composé de formule (I), dans laquelle $X_3$ et/ou Y représentent un groupe carboxyle, au moyen de carbonyldiimidazole, puis on traite l'imidazolide intermédiaire ainsi obtenu in situ par un amide de formules $R_3R_4NH$ ou $R_9R_{10}$, respectivement, dans lesquelles $R_3$, $R_4$, $R_9$ et $R_{10}$ sont tels que définis ci-dessus.

## Ansprüche

**Patentansprüche für die Vertragsstaaten : BE, CH, DE, FR, GB, IT, LI, LU, NL, SE**

1. Verbindungen der allgemeinen Formel (I)

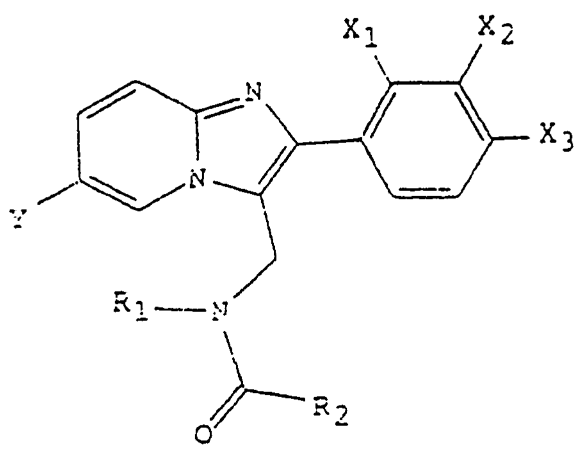

(I)

wobei

$R_1$ ein Wasserstoffatom oder eine lineare oder verzweigte $C_1$-$C_4$-Alkylgruppe oder eine Benzylgruppe darstellt,

$R_2$ eine lineare oder verzeigte $C_1$-$C_6$-Alkylgruppe, eine Cyclohexyl-, Trichlormethyl-, Propen-1-yl-, Allyl-, Phenyl-, 4-Chlorphenyl- oder Benzyl-Gruppe darstellt, oder aber

$R_1$ und $R_2$ gemeinsam eine aliphatische $C_3$-$C_5$-Kette darstellen, und entweder

a) $X_1$ und $X_2$ jeweils ein Wasserstoffatom darstellen,

$X_3$ ein Halogenatom darstellt oder eine $C_1$-$C_3$-Alkylgruppe, eine Methylthiogruppe, eine Trifluoromethylgruppe, eine unter Umständen veresterte Carboxygruppe (der Formel COOR, wobei R Wasserstoff oder ein

$C_1$-$C_6$-Alkyl darstellt), eine Cyanogruppe, eine unter Umständen mono- oder dialkylierte Aminocarbonylgruppe (der Formel $CONR_3R_4$ wobei $R_3$ und $R_4$ unabhängig voneinander jeweils Wasserstoff oder ein $C_1$-$C_4$-Alkyl oder gemeinsam eine Kette der Formel $(CH_2)_2$-Z-$(CH_2)_2$ darstellen, wobei Z eine Bindung oder ein Sauerstoffatom oder ein Schwefelatom oder eine Gruppe der Formel $CH_2$, NH oder N·($C_1$-$C_4$-alkyliert) darstellt), eine Alkylaminogruppe (der Formel $NHR_5$ wobei $R_5$ eine $C_2$-$C_6$-Alkylgruppe darstellt) oder eine Dialkylaminogruppe (der Formel $NR_6R_7$ wobei $R_6$ und $R_7$ unabhängig voneinander jeweils ein $C_2$-$C_6$-Alkyl oder gemeinsam eine Kette der Formel $(CH_2)_2$-Z-$(CH_2)_2$ darstellen, wobei Z eine Bindung oder ein Sauerstoffatom oder ein Schwefelatom oder eine Gruppe der Formel $CH_2$, NH oder N ($C_1$-$C_4$-alkyliert) darstellt), und

Y eine Methylgruppe darstellt, eine Trifluoromethylgruppe, eine unter Umständen veresterte Carboxygruppe (der Formel $COOR_8$ wobei $R_8$ Wasserstoff oder ein $C_1$-$C_6$-Alkyl darstellt), eine Cyanogruppe, eine unter Umständen mono- oder dialkylierte Aminocarbonylgruppe (der Formel $CONR_9R_{10}$ wobei $R_9$ und $R_{10}$ unabhängig voneinander jeweils Wasserstoff oder ein $C_1$-$C_4$-Alkyl darstellen) oder eine unter Umständen mono- oder dialkylierte Aminogruppe (der Formel $NR_{11}R_{12}$ wobei $R_{11}$ und $R_{12}$ unabhängig voneinander jeweils Wasserstoff oder ein $C_1$-$C_4$-Alkyl darstellen),

wobei im Fall (a) jene Verbindungen der Formel (I) ausgeschlossen sein sollen, bei welchen $X_3$ ein Halogenatom oder eine Alkyl- oder Methylthiogruppe und Y eine Methylgruppe darstellen, oder aber

b) $X_1$, $X_2$ und $X_3$ unabhängig voneinander jeweils Wasserstoff, ein Halogen oder eine $C_1$-$C_4$-Alkylgruppe, eine Methoxy-, Methylthio-, Äthylthio-, Methylsulfonyl-, Nitro-, Amino-, Methylamino-, Dimethylamino-, Acetylamino- oder Diacetylaminogruppe darstellen und

Y eine Methylgruppe darstellt,

wobei im Fall (b) jene Verbindungen der Formel (I) ausgeschlossen sein sollen, bei welchen $X_1$ und $X_2$ jeweils ein Wasserstoffatom darstellen,

sowie ihre Additionssalze mit in der Pharmakologie verwendbaren Säuren.

2. Verbindungen nach Anspruch 1, dadurch gekennzeichnet, daß in der Formel (I) $R_1$ ein Wasserstoffatom oder eine Methylgruppe, und $R_2$ eine Propyl- oder Isobutylgruppe darstellen.

3. Verfahren zur Herstellung von Verbindungen nach Anspruch 1, dadurch gekennzeichnet, daß ein 2-Aminopyridin der Formel (II)

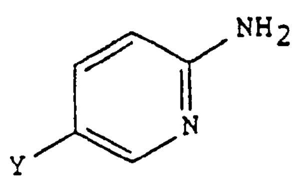

(II)

(wobei Y wie oben definiert ist) mit einem 2-Brom-äthanon der Formel (III)

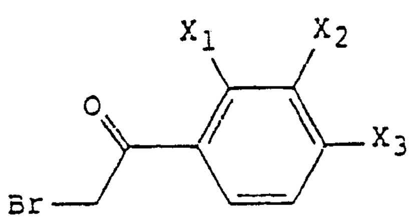

(III)

(wobei $X_1$, $X_2$ und $X_3$ wie oben definiert sind) in der Hitze in einem geeigneten protischen Lösungsmittel zur Reaktion gebracht wird,

und hierauf das so erhaltene Imidazo[1,2-a]pyridin der Formel (IV)

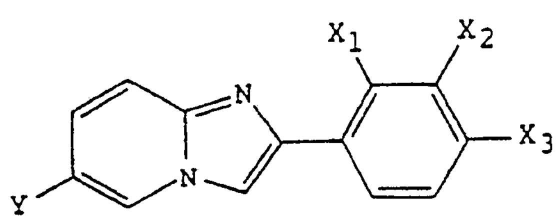

(IV)

einer Hydroxymethylierung mit Formaldehyd in einem Lösungsmittel vom Carbonsäuretyp unterworfen wird,

hierauf der so erhaltene Alkohol der Formel (V)

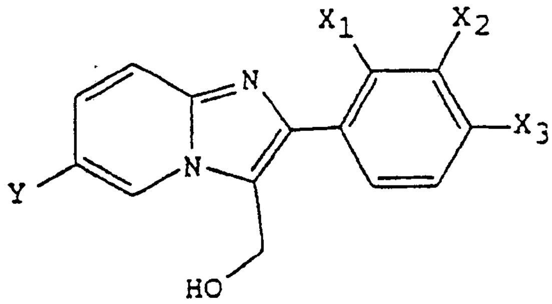

(V)

mit einem Nitril der Formel $R_2$-CN (wobei $R_2$ wie oben definiert ist) in saurem Milieu und bei einer Temperatur von 0 bis 150°C zur Reaktion gebracht wird,

hierauf nach Hydrolyse des Reaktionsprodukts falls gewünScht die so erhaltene verbindung der Formel (Ia)

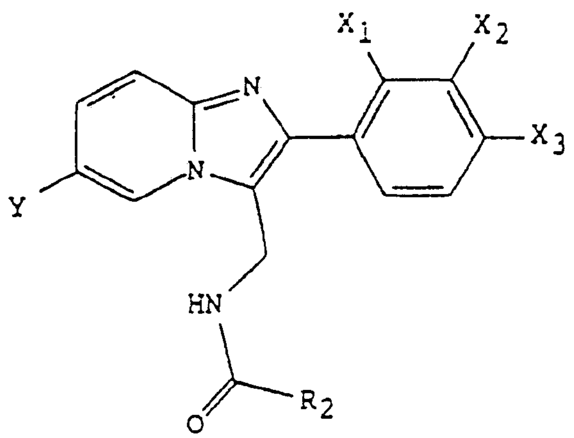

(Ia)

einer Alkylierung oder einer Benzylierung durch ein Halogenid der Formel $R_1$-hal (wobei $R_1$ wie oben definiert ist) in einem Lösungsmittel und in Anwesenheit eines Alkalimetallhydrids unterworfen wird.

4. Verfahren nach Anspruch 3, dadurch gekennzeichnet, daß nach der Reaktion zwischen den Verbindungen der Formel (II) und (III) das so erhaltene Imidazo[1,2-a]pyridin der Formel (IV) mit einem Amid der Formel

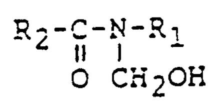

(wobei $R_1$ und $R_2$ wie oben definiert sind) bei Raumtemperatur und in Anwesenheit von konzentrierter Schwefelsaure zur Reaktion gebracht wird.

5. Verfahren nach Anspruch 3, dadurch gekennzeichnet, daß nach der Reaktion zwischen den Verbindungen der Formel (II) und (III) eine Verbindung der Formel (Ia) hergestellt wird, indem das Imidazo[1,2-a]pyridin der Formel (IV) mit einem Nitril der Formel $R_2$-CN und mit Paraformaldehyd in der Hitze in essigsaurem Milieu und in Anwesenheit von Schwefelsäure zur Reaktion gebracht wird.

6. Verfahren nach Anspruch 3, dadurch gekennzeichnet, daß, um eine Verbindung der Formel (I) zu erhalten, bei welcher $X_3$ und /oder Y eine Cyanogruppe darstellen, eine Verbindung der Formel (I), bei der $X_3$ und-/oder Y ein Jodatom darstellen, mit einem Metallcyanid in einem inerten Lösungsmittel zur Reaktion gebracht wird.

7. Verfahren nach Anspruch 3, dadurch gekennzeichnet, daß, um eine Verbindung der Formel (I) zu erhalten, bei welcher $X_3$ und/oder Y eine Carboxylatgruppe darstellen, eine Verbindung der Formel (I), bei der $X_3$ und/oder Y eine Cyanogruppe darstellen, mit gasförmigem Chlorwasserstoff in einem Alkohol der Formel ROH oder $R_8$OH zur Reaktion gebracht wird, wobei R und $R_8$ wie oben definiert sind.

8. Verfahren nach Anspruch 3, dadurch gekennzeichnet, daß, um eine Verbindung der Formel (I) zu erhalten, bei welcher $X_3$ und/oder Y eine Carboxyl- oder Carboxamidgruppe darstellen, eine Verbindung der Formel (I), bei der $X_3$ und/oder Y eine Cyanogruppe darstellen, im sauren Milieu bei einer Temperatur von 60 bis 120°C hydrolysiert wird.

9. Verfahren nach Anspruch 3, dadurch gekennzeichnet, daß, um eine Verbindung der Formel (I) zu erhalten, bei welcher $X_3$ und/oder Y eine Carboxamidgruppe darstellen, eine Verbindung der Formel (I), bei der $X_3$ und/oder Y eine Carboxylgruppe darstellen, mit Carbonyldiimidazol amidiert wird und das so erhaltene Imidazolid-Zwischenprodukt in situ mit einem Amid der Formel $R_3R_4NH$ bzw. $R_9R_{10}$ behandelt wird, wobei $R_3$, $R_4$, $R_9$ und $R_{10}$ wie oben definiert sind.

10. Arzneimittel, dadurch gekennzeichnet, daß es aus einer Verbindung nach einem der Ansprüche 1 und 2 besteht.

11. Pharmazeutische Zusammensetzung, dadurch gekennzeichnet, daß sie eine Verbindung nach einem der Ansprüche 1 und 2 gemeinsam mit geeigneten Hilfsstoffen enthält.

**Patentansprüche für die Vertragsstaaten AT, ES, GR**

1. Verfahren zur Herstellung von Verbindungen der allgemeinen Formel (I)

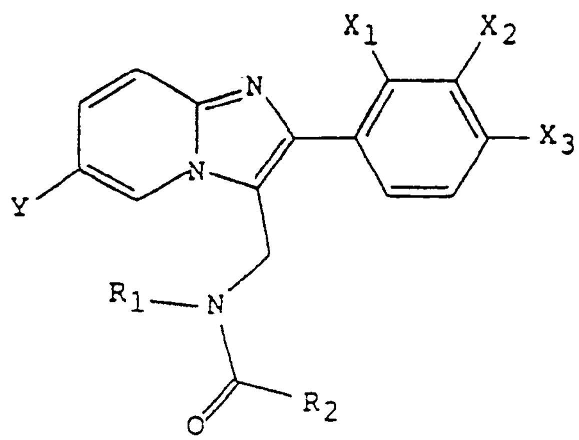

(I)

wobei

$R_1$ ein Wasserstoffatom oder eine lineare oder verzweigte $C_1$-$C_4$-Alkylgruppe oder eine Benzylgruppe darstellt,

$R_2$ eine lineare oder verzeigte $C_1$-$C_6$-Alkylgruppe, eine Cyclohexyl-, Trichlormethyl-, Propen-1-yl-, Allyl-, Phenyl-, 4-Chlorphenyl- oder Benzyl-Gruppe darstellt, oder aber

$R_1$ und $R_2$ gemeinsam eine aliphatische $C_3$-$C_5$-Kette darstellen,

und entweder

a) $X_1$ und $X_2$ jeweils ein Wasserstoffatom darstellen, $X_3$ ein Halogenatom darstellt oder eine $C_1$-$C_3$-Alkylgruppe, eine Methylthiogruppe, eine Trifluoromethylgruppe, eine unter Umständen veresterte Carboxygruppe (der Formel COOR, wobei R Wasserstoff oder ein $C_1$-$C_6$-Alkyl darstellt), eine Cyanogruppe, eine unter Umständen mono- oder dialkylierte Aminocarbonylgruppe (der Formel $CONR_3R_4$ wobei $R_3$ und $R_4$ unabhängig voneinander jeweils Wasserstoff oder ein $C_1$-$C_4$-Alkyl oder gemeinsam eine Kette der Formel $(CH_2)_2$-Z-$(CH_2)_2$ darstellen, wobei Z eine Bindung oder ein Sauerstoffatom oder ein Schwefelatom oder eine Gruppe der Formel $CH_2$, NH oder N ($C_1$-$C_4$-alkyliert) darstellt), eine Alkylaminogruppe (der Formel $NHR_5$ wobei $R_5$ eine $C_2$-$C_6$-Alkylgruppe darstellt) oder eine Dialkylaminogruppe (der Formel $NR_6R_7$ wobei $R_6$ und $R_7$ unabhängig voneinander jeweils ein $C_2$-$C_6$-Alkyl oder gemeinsam eine Kette der Formel $(CH_2)_2$-Z-$(CH_2)_2$ darstellen, wobei Z eine Bindung oder ein Sauerstoffatom oder ein Schwefelatom oder eine Gruppe der Formel $CH_2$, NH oder N ($C_1$-$C_4$-alkyliert) darstellt), und

Y eine Methylgruppe darstellt, eine Trifluoromethylgruppe, eine unter Umständen veresterte Carboxygruppe (der Formel $COOR_8$ wobei $R_8$ Wasserstoff oder ein $C_1$-$C_6$-Alkyl darstellt), eine Cyanogruppe, eine unter Umständen mono- oder dialkylierte Aminocarbonylgruppe (der Formel $CONR_9R_{10}$ wobei $R_9$ und $R_{10}$ unabhängig voneinander jeweils Wasserstoff oder ein $C_1$-$C_4$-Alkyl darstellen) oder eine unter Umständen mono- oder dialkylierte Aminogruppe (der Formel $NR_{11}R_{12}$ wobei $R_{11}$ und $R_{12}$ unabhängig voneinander jeweils Wasserstoff oder ein $C_1$-$C_4$-Alkyl darstellen),

wobei im Fall (a) jene Verbindungen der Formel (I) ausgeschlossen sein sollen, bei welchen $X_3$ ein Halo-

genatom oder eine Alkyl- oder Methylthiogruppe und Y eine Methylgruppe darstellen, oder aber

b) $X_1$, $X_2$ und $X_3$ unabhängig voneinander jeweils Wasserstoff, ein Halogen oder eine $C_1$-$C_4$-Alkylgruppe, eine Methoxy-, Methylthio-, Äthylthio-, Methylsulfonyl-, Nitro-, Amino-, Methylamino-, Dimethylamino-, Acetylamino- oder Diacetylaminogruppe darstellen

und

Y eine Methylgruppe darstellt,

wobei im Fall (b) jene Verbindungen der Formel (I) ausgeschlossen sein sollen, bei welchen $X_1$ und $X_2$ jeweils ein Wasserstoffatom darstellen,

das Verfahren dadurch gekennzeichnet, daß ein 2-Aminopyridin der Formel (II)

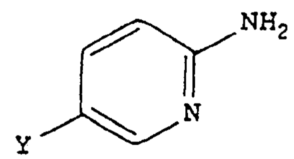

(II)

(wobei Y wie oben definiert ist) mit einem 2-Brom-äthanon der Formel (III)

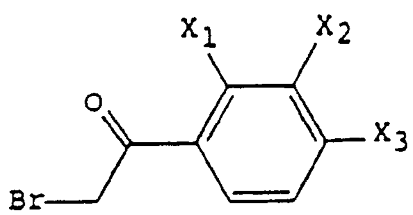

(III)

(wobei $X_1$, $X_2$ und $X_3$ wie oben definiert sind) in der Hitze in einem geeigneten protischen Lösungsmittel zur Reaktion gebracht wird,

und hierauf das so erhaltene Imidazo[1,2-a]pyridin der Formel (IV)

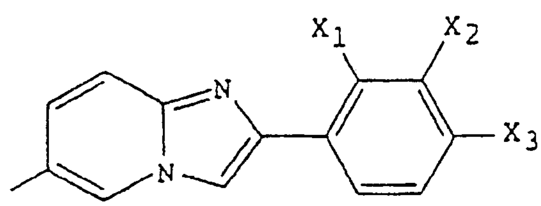

(IV)

einer Hydroxymethylierung mit Formaldehyd in einem Lösungsmittel vom Carbonsäuretyp unterworfen wird,

hierauf der so erhaltene Alkohol der Formel (V)

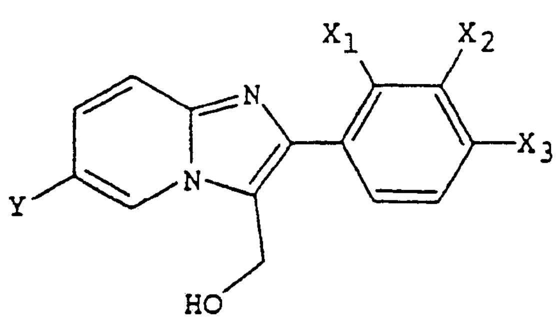

(V)

mit einem Nitril der Formel $R_2$-CN (wobei $R_2$ wie oben definiert ist) in saurem Milieu und bei einer Temperatur von 0 bis 150°C zur Reaktion gebracht wird,

hierauf nach Hydrolyse des Reaktionsprodukts falls gewünscht die so erhaltene Verbindung der Formel (Ia)

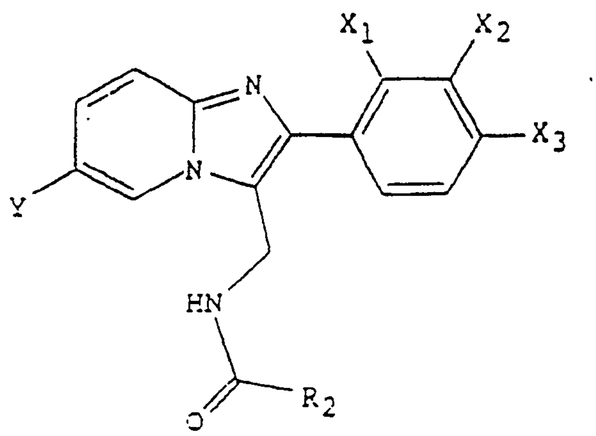

(Ia)

einer Alkylierung oder einer Benzylierung durch ein Halogenid der Formel $R_1$-hal (wobei $R_1$ wie oben definiert ist) in einem Lösungsmittel und in Anwesenheit eines Alkalimetallhydrids unterworfen wird.

2. Verfahren nach Anspruch 1, dadurch gekennzeichnet, daß nach der Reaktion zwischen den Verbindungen der Formel (II) und (III) das so erhaltene Imidazo[1,2-a]pyridin der Formel (IV) mit einem Amid der Formel

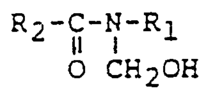

(wobei $R_1$ und $R_2$ wie oben definiert sind) bei Raumtemperatur und in Anwesenheit von konzentrierter Schwefelsäure zur Reaktion gebracht wird.

3. Verfahren nach Anspruch 1, dadurch gekennzeichnet, daß nach der Reaktion zwischen den Verbindungen der Formel (II) und (III) eine Verbindung der Formel (Ia) hergestellt wird, indem das Imidazo[1,2-a]pyridin der Formel (IV) mit einem Nitril der Formel $R_2$-CN und mit Paraformaldehyd in der Hitze in essigsaurem Milieu und in Anwesenheit von Schwefelsäure zur Reaktion gebracht wird.

4. Verfahren nach Anspruch 1, dadurch gekennzeichnet, daß, um eine Verbindung der Formel (I) zu erhalten, bei welcher $X_3$ und/oder Y eine Cyanogruppe darstellen, eine Verbindung der Formel (I), bei der $X_3$ und /oder Y ein Jodatom darstellen, mit einem Metallcyanid in einem inerten Lösungsmittel zur Reaktion gebracht wird.

5. Verfahren nach Anspruch 1, dadurch gekennzeichnet, daß, um eine Verbindung der Formel (I) zu erhalten, bei welcher $X_3$ und/oder Y eine Carboxylatgrugpe darstellen, eine Verbindung der Formel (I), bei der $X_3$ und/oder Y eine Cyanogrugpe darstellen, mit gasförmigem Chlorwasserstoff in einem Alkohol der Formel ROH oder $R_8$OH zur Reaktion gebracht wird, wobei R und $R_8$ wie oben definiert sind.

6. Verfahren nach Anspruch 1, dadurch gekennzeichnet, daß, um eine Verbindung der Formel (I) zu erhalten, bei welcher $X_3$ und/oder Y eine Carboxyl- oder Carboxamidgrugge darstellen, eine Verbindung der Formel (I), bei der $X_3$ und/oder Y eine Cyanogruppe darstellen, im sauren Milieu bei einer Temperatur von 60 bis 120°C hydrolysiert wird.

7. Verfahren nach Anspruch 1, dadurch gekennzeichnet, daß, um eine Verbindung der Formel (I) zu erhalten, bei welcher $X_3$ und/oder Y eine Carboxamidgrugpe darstellen, eine Verbindung der Formel (I), bei der $X_3$ und/oder Y eine Carboxylgruppe darstellen, mit Carbonyldiimidazol amidiert wird und das so erhaltene Imidazolid-Zwischengrodukt in situ mit einem Amid der Formel $R_3R_4NH$ bzw. $R_9R_{10}$ behandelt wird, wobei $R_3$, $R_4$, $R_9$ und $R_{10}$ wie oben definiert sind.

## Claims

### Claims for the Contracting States : BE, CH, DE, FR, GB, IT, LI, LU, NL, SE

1. Compounds of general formula (I)

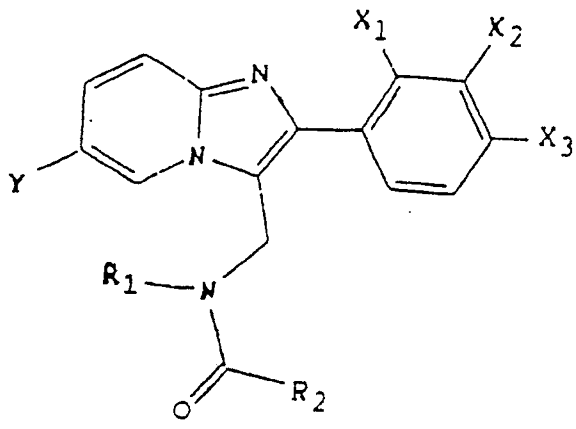

(I)

in which

$R_1$ denotes a hydrogen atom or a linear or branched $C_1$-$C_4$ alkyl group or a benzyl group,

$R_2$ denotes a linear or branched $C_1$-$C_6$ alkyl group or a cyclohexyl, trichloromethyl, 1-propenyl, allyl, phenyl, 4-chlorophenyl or benzyl group,

or alternatively

$R_1$ and $R_3$ together denote a $C_3$-$C_5$5 aliphatic chain, and either

a) $X_1$ and $X_2$ each denote a hydrogen atom,

$X_3$ denotes a halogen atom or a $C_1$-$C_3$ alkyl group, a methylthio group, a trifluoromethyl group, an optionally esterified carboxy group (of formula COOR in which R denotes hydrogen or a $C_1$-$C_6$ alkyl), a cyano group, an aminocarbonyl group which is optionally mono- or dialkylated (of formula $CONR_3R_4$ in which $R_3$ and $R_4$ each denote, independently of one another, hydrogen or a $C_1$-$C_4$ alkyl or together denote a chain of formula $(CH_2)_2$-Z-$(CH_2)_2$ in which Z denotes a bond or an oxygen or sulphur atom or a group of formula $CH_2$, NH or N($C_1$-$C_4$ alkyl)), an alkylamino group (of formula $NHR_5$ in which $R_5$ denotes a $C_2$-$C_6$ alkyl) or a dialkylamino group (of formula $NR_6R_7$ in which $R_6$ and $R_7$ each denote, independently of one another, a $C_2$-$C_6$ alkyl or together denote a chain of formula $(CH_2)_2$-Z-$(CH_2)_2$ in which Z denotes a bond or an oxygen or sulphur atom or a group of formula $CH_2$, NH or N($C_1$-$C_4$ alkyl)), and

Y denotes a methyl group, a trifluoromethyl group, an optionally esterified carboxy group (of formula $COO_8$ in which $R_8$ denotes hydrogen or a $C_1$-$C_6$ alkyl), a cyano group, an aminocarbonyl group which is optionally mono- or dialkylated (of formula $CON_9R_{10}$ in which $R_9$ and $R_{10}$ each denote, independently of one another, hydrogen or a $C_1$-$C_4$ alkyl), or an amino group which is optionally mono- or dialkylated (of formula $NR_{11}R_{12}$ in which $R_{11}$ and $R_{12}$ each denote, independently of one another, hydrogen or a $C_1$-$C_4$ alkyl), with the exclusion, in the case (a), of the compounds of formula (I) in which $X_3$ denotes a halogen atom or an alkyl or methylthio group and Y denotes a methyl group, or alternatively

b) $X_1$, $X_2$ and $X_3$ each denote, independently of one another, hydrogen, a halogen or a $C_1$-$C_4$ alkyl group or a methoxy, methylthio, ethylthio, methylsulphonyl, nitro, amino, methylamino, dimethylamino, acetylamino or diacetylamino group, and

Y denotes a methyl group,

with the exclusion, in the case (b), of the compounds in the formula (I) of which $X_1$ and $X_2$ each denote a hydrogen atom, as well as their addition salts with acids which are pharmacologically acceptable.

2. Compounds according to Claim 1, characterized in that, in the formula (I), $R_1$ denotes a hydrogen atom or a methyl group, and $R_2$ denotes a propyl or isobutyl group.

3. Process for preparing compounds according toClaim 1, characterized in that a 2-aminopyridine of formula (II)

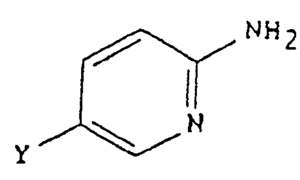

(II)

(in which Y is as defined above), is reacted with a 2-bromoethanone of formula (III)

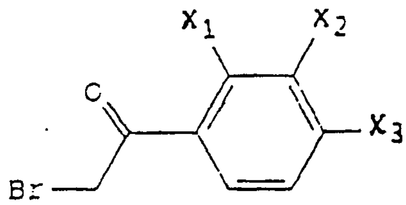

(III)

(in which $X_1$, $X_2$ and $X_3$ are as defined above), in the heated state and in a protic solvent, the imidazo[1,2-a]pyridine of formula (IV) thereby obtained

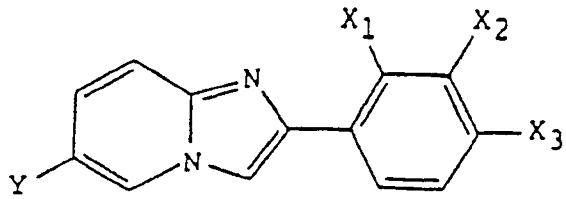

(IV)

is then subjected to a hydroxymethylation by means of formaldehyde in a solvent of the carboxylic acid type, the alcohol of formula (V) thereby obtained

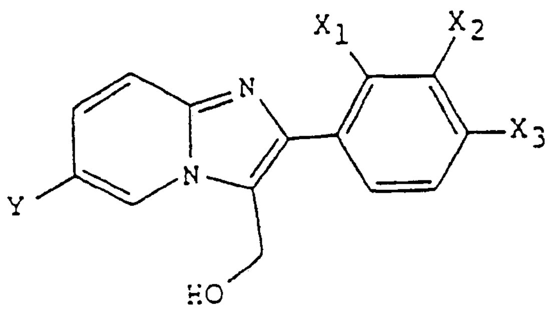

(V)

is then reacted with a nitrile of formula $R_2$-CN (in which $R_2$ is as defined above), in acid medium and at a temperature of 0 to 150°C,
and then, after hydrolysis of the reaction product, and if so desired, the compound of formula (Ia) thereby obtained

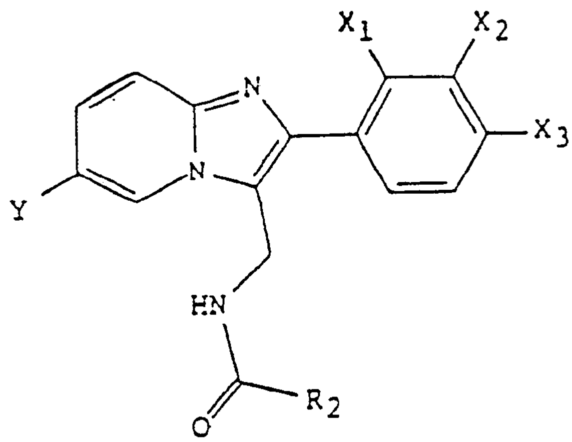

(Ia)

is subjected to an alkylation or a benzylation with a halide of formula $R_1$-hal (in which $R_1$ is an defined above), in a solvent and in the presence of an alkali metal hydride.

4. Process according to Claim 3, characterized in that, after the reaction between the compounds of formulae (II) and (III), the imidazo[1,2-a]pyridine of formula (IV) thereby obtained is reacted with an amide of formula

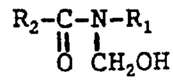

(in which $R_1$ and $R_2$ are as defined above), at room temperature and in the presence of concentrated sulphuric acid.

5. Process according to Claim 3, characterized in that, after the reaction between the compounds of formulae (II) and (III), a compound of formula (Ia) is prepared by reacting the imidazo[1,2-a]pyridine of formula (IV) with a nitrile of formula $R_2$-CN and with paraformaldehyde, in the heated state, in acetic acid medium and in the presence of sulphuric acid.

6. Process according to Claim 3, characterized in that, to obtain a compound of formula (I) in which $X_3$ and/or Y denote a cyano group, a compound of formula (I), in which $X_3$ and/or Y denote an iodine atom, is reacted with a metal cyanide in an inert solvent.

7. Process according to Claim 3, characterized in that, to obtain a compound of formula (I) in which $X_3$ and/or Y denote a carboxylate group, a compound of formula (I), in which $X_3$ and/or Y denote a cyano group, is reacted with gaseous hydrochloric acid in an alcohol of formula ROH or $R_8$OH, R and $R_8$ being as defined above.

8. Process according to Claim 3, characterized in that, to obtain a compound of formula (I) in which $X_3$ and/or Y denote a carboxyl or carboxamide group, a compound of formula (I), in which $X_3$ and/or Y denote a cyano group, is hydrolysed in acid medium, at a temperature of 60 to 120°C.

9. Process according to Claim 3, characterized in that, to obtain a compound of formula (I) in which $X_3$ and/or Y denote a carboxamide group, a compound of formula (I), in which $X_3$ and/or Y denote a carboxyl group, is amidated by means of carbonyldiimidazole and the intermediate imidazolide thereby obtained is treated in situ with an amide of formulae $R_3R_4$NH or $R_9R_{10}$, respectively, in which $R_3$, $R_4$, $R_9$ and $R_{10}$ are as defined above.

10. Medicinal product, characterized in that it consists of a compound according to one of Claims 1 and 2.

11. Pharmaceutical composition, characterized in that it contains a compound according to one of Claims 1 and 2, in combination with a suitable excipient.

**Claims for the Contracting States : AT, ES, GR**

1. Process for preparing compounds of general formula (I)

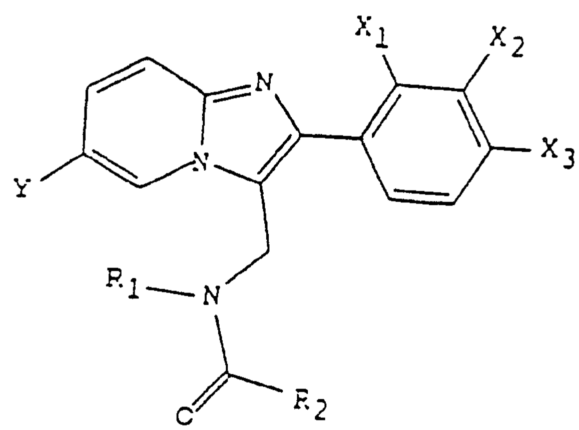

in which

$R_1$ denotes a hydrogen atom or a linear or branched $C_1$-$C_4$ alkyl group or a benzyl group,

$R_2$ denotes a linear or branched $C_1$-$C_6$ alkyl group or a cyclohexyl, trichloromethyl, 1-propenyl, allyl, phenyl, 4-chlorophenyl or benzyl group,

or alternatively

$R_1$ and $R_2$ together denote a $C_3$-$C_5$ aliphatic chain, and either

a) $X_1$ and $X_2$ each denote a hydrogen atom,

$X_3$ denotes a halogen atom or a $C_1$-$C_3$ alkyl group, a methylthio group, a trifluoromethyl group, an optionally esterified carboxy group (of formula COOR in which R denotes hydrogen or a $C_1$-$C_6$ alkyl), a cyano group, an

aminocarbonyl group which is optionally mono- or dialkylated (of formula $CONR_3R_4$ in which $R_3$ and $R_4$ each denote, independently of one another, hydrogen or a $C_1$-$C_4$ alkyl or together denote a chain of formula $(CH_2)_2$-Z-$(CH_2)_2$ in which Z denotes a bond or an oxygen or sulphur atom or a group of formula $CH_2$, NH or N($C_1$-$C_4$ alkyl)), an alkylamino group (of formula $NHR_5$ in which $R_5$ denotes a $C_2$-$C_6$ alkyl) or a dialkylamino group (of formula $NR_6R_7$ in which $R_6$ and $R_7$ each denote, independently of one another, a $C_2$-$C_6$ alkyl or together denote a chain of formula $(CH_2)_2$-Z-$(CH_2)_2$ in which Z denotes a bond or an oxygen or sulphur atom or a group of formula $CH_2$, NH or N($C_1$-$C_4$4 alkyl)), and

Y denotes a methyl group, a trifluoromethyl group, an optionally esterified carboxy group (of formula $COOR_8$ in which $R_8$ denotes hydrogen or a $C_1$-$C_6$ alkyl), a cyano group, an aminocarbonyl group which is optionally mono-or dialkylated (of formula $CONR_9R_{10}$ in which $R_9$ and $R_{10}$ each denote, independently of one another, hydrogen or a $C_1$-$C_4$ alkyl), or an amino group which is optionally mono- or dialkylated (of formula $NR_{11}R_{12}$ in which $R_{11}$ and $R_{12}$ each denote, independently of one another, hydrogen or a $C_1$-$C_4$ alkyl), with the exclusion, in the case (a), of the compounds of formula (I) in which $X_3$ denotes a halogen atom or an alkyl or methylthio group and Y denotes a methyl group,

or alternatively

b) $X_1$, $X_2$ and $X_3$ each denote, independently of one another, hydrogen, a halogen or a $C_1$-$C_4$ alkyl group or a methoxy, methylthio, ethylthio, methylsulphonyl, nitro, amino, methylamino, dimethylamino, acetylamino or diacetylamino group, and

Y denotes a methyl group,

with the exclusion, in the case (b), of the compounds in the formula (I) of which $X_1$and $X_2$ each denote a hydrogen atom,

which process is characterized in that a 2-aminopyridine of formula (II)

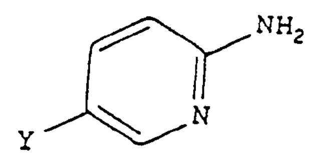

(in which Y is as defined above), is reacted with a 2-bromoethanone of formula (III)

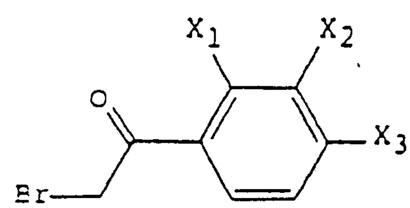

(in which $X_1$, $X_2$ and $X_3$ are as defined above), in the heated state and in a protic solvent, the imidazo[1,2-a]pyridine of formula (IV) thereby obtained

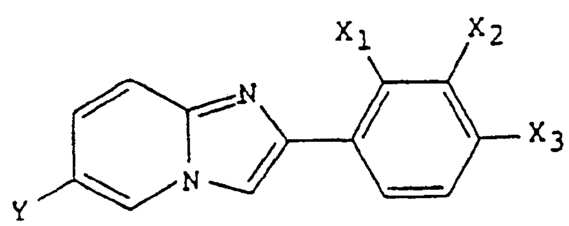

is then subjected to a hydroxymethylation by means of formaldehyde in a solvent of the carboxylic acid the, the alcohol of formula (V) thereby obtained

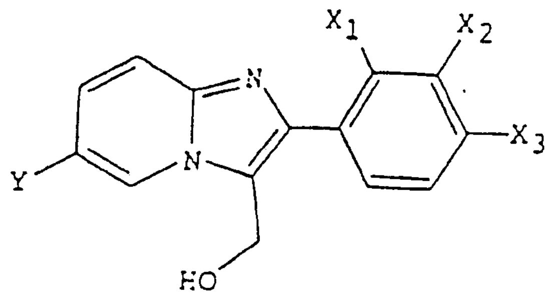

is then reacted with a nitrile of formula $R_2$-CN (in which

$R_2$ is as defined above), in acid medium and at a temperature of 0 to 150°C,

and then, after hydrolysis of the reaction product, and if so desired, the compound of formula (Ia) thereby obtained

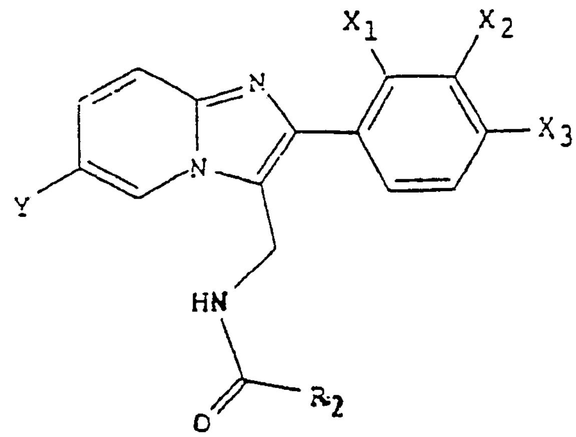

is subjected to an alkylation or a benzylation with a halide of formula $R_1$-hal (in which $R_1$ is as defined above), in a solvent and in the presence of an alkali metal hydride.

2. Process according to Claim 1, characterized in that, after the reaction between the compounds of formulae (II) and (III), the imidazo[1,2-a]pyridine of formula (IV) thereby obtained is reacted with an amide of formula

$$R_2-\underset{\underset{O}{\|}}{C}-\underset{\underset{CH_2OH}{|}}{N}-R_1$$

(in which $R_1$ and $R_2$ are as defined above), at room temperature and in the presence of concentrated sulphuric acid.

3. Process according to Claim 1, characterized in that, after the reaction between the compounds of formulae (II) and (III), a compound of formula (Ia) is prepared by reacting the imidazo[1,2-a]pyridine of formula (IV) with a nitrile of formula $R_2$-CN and with paraformaldehyde, in the heated state, in acetic acid medium and in the presence of sulphuric acid.

4. Process according to Claim 1, characterized in that, to obtain a compound of formula (I) in which $X_3$ and/or Y denote a cyano group, a compound of formula (I), in which $X_3$ and/or Y denote an iodine atom, is reacted with a metal cyanide in an inert solvent.

5. Process according to Claim 1, characterized in that, to obtain a compound of formula (I) in which $X_3$ and/or Y denote a carboxylate group, a compound of formula (I), in which $X_3$ and/or Y denote a cyano group, is reacted with gaseous hydrochloric acid in an alcohol of formula ROH or $R_8$OH, R and $R_8$ being as defined above.

6. Process according to Claim 1, characterized in that, to obtain a compound of formula (I) in which $X_3$ and/or Y denote a carboxyl or carboxamide group, a compound of formula (I), in which $X_3$ and/or Y denote a cyano group, is hydrolysed in acid medium, at a temperature of 60 to 120°C.

7. Process according to Claim 1, characterized in that, to obtain a compound of formula (I) in which $X_3$ and/or Y denote a carboxamide group, a compound of formula (I), in which $X_3$ and/or Y denote a carboxyl group, is

amidated by means of carbonyldiimidazole and the intermediate imidazolide thereby obtained is treated in situ with an amide of formulae $R_3R_4NH$ or $R_9R_{10}$, respectively, in which $R_3$, $R_4$, $R_9$ and $R_{10}$ are as defined above.